# EUROPEAN PATENT APPLICATION

(11) **EP 4 617 288 A1**
(43) Date of publication of application: **17.09.2025**
(21) Application number: 23888778.0
(22) Date of filing: 09.11.2023
(51) Int. Cl.: C07K 19/00, A61K 38/16, A61K 47/61, A61P 25/18, A61P 25/24, A61P 25/28, C07K 14/47, C12N 15/11, C12N 15/12

(54) **GLYCOSYLATED NEUROPEPTIDE DERIVATIVE INCLUDING NEUROPEPTIDE SEQUENCE AND SUGAR CHAIN, PHARMACEUTICAL COMPOSITION, TRANSNASAL/NASAL DRIP FORMULATION, AND USE OF GLYCOSYLATED NEUROPEPTIDE DERIVATIVE**

(30) Priority: 10.11.2022 JP 2022180380
(71) Applicant: TOKYO UNIVERSITY OF SCIENCE FOUNDATION, Tokyo 162-8601 (JP)
(72) Inventor: YAMASHITA, Chikamasa, Tokyo 162-8601 (JP)
(74) Representative: Berggren Oy
(86) International application number: PCT/JP2023/040454
(87) International publication number: WO 2024/101433

(57) **Abstract**

A glycosylated neuropeptide derivative, comprising: a neuropeptide sequence; a central-nerve migration accelerating sequence that includes a cell-penetration accelerating sequence and an endosomal-escape accelerating sequence; and a glycosylation molecule that includes a sugar chain.

## Description

### [Technical Field]

The present invention relates to a glycosylated neuropeptide derivative including a neuropeptide sequence and a sugar chain, a pharmaceutical composition, an intranasal/nasal drop formulation, and a use of the glycosylated neuropeptide derivative.

### [Background Art]

Central nervous system diseases such as Alzheimer's disease, vascular dementia and amyotrophic lateral sclerosis are known to have a high degree of unmet medical needs, i.e., diseases with a low degree of medical satisfaction and an insufficient variety of effective drugs, and development of new drugs is required. On the other hand, a certain degree of therapeutic effects has been achieved by the use of low-molecular drugs for treating depression. However, since approximately 30-40% of patients exhibit treatment resistivity to existing anti-depressant drugs, development of drugs with a new medicinal mechanism is desired.

In view of the foregoing, neuropeptides, which express a different medical mechanism from that of low-molecular drugs, are attracting attention as possible alternative drugs. For example, it is known that glucagon-like peptide-1 (GLP-1), a peptide derived from a proglucagon and formed of 37 amino acid residues, and glucagon-like peptide-2 (GLP-2), a peptide derived from a proglucagon and formed of 33 amino acid residues, bind to a G-protein-coupled receptor (GPCR) and activate the signaling transduction.

Regarding the pharmacological activity of GLP-1 (activity types: 7-37 and 7-36 amide) in the brain, there have been reports of an effect of alleviating learning disorders (for example, Non-Patent Documents 1 and 2).

Regarding the pharmacological activity of GLP-2 in the brain, there have been reports of effects of anti-depressant activity even in treatment-resistant depression model animals, lowering blood pressure, and alleviating learning disorders (for example, Non-Patent Documents 3 to 9).

Further, it has been reported that neuromedin U (NmU), a peptide formed of 23 amino acid residues, binds to a GPCR in the brain and exerts an effect of alleviating learning disorders (for example, Non-Patent Document 10).

In addition, research and development have been conducted on centrally-acting peptides such as enkephalin (5 amino acid residues), pasireotide (5 amino acid residues), ocretide (7 amino acid residues), lanreotide (7 amino acid residues), oxytocin (9 amino acid residues), somatostatin-14 (14 amino acid residues), dynorphin (17 amino acid residues), somatostatin-28 (28 amino acid residues), ghrelin (28 amino acid residues), orexin B (28 amino acid residues), galanin (30 amino acid residues), β-endorphin (31 amino acid residues), orexin A (33 amino acid residues), neuropeptide Y (36 amino acid residues), insulin (51 amino acid residues), galanin-like peptide (60 amino acid residues), insulin-like growth factor-1 (70 amino acid residues), nerve growth factor (118 amino acid residues) and leptin (166 amino acid residues).

One significant factor behind a high degree of unmet medical needs in central nervous system diseases is difficulty in delivery of a drug to a target region due to tight cell-to-cell conjugation represented by blood-brain barrier (BBB), which makes a delivery route for a drug from blood to a brain tissue extremely restricted. For example, it is not possible for 100% of large molecules with a size of over 500 Da or not less than 98% of molecules smaller than the same to penetrate the BBB (Non-Patent Document 11). Therefore, a pharmaceutical test for a drug in central nervous system diseases is performed by way of intraventricular administration, i.e., injecting a drug directly into the brain. However, the application of intraventricular injection to clinical practice is unrealistic due to its highly invasive nature. In view of the foregoing, intranasal administration has been attracting attention in which a drug is administered to the nasal cavity, which is anatomically located in the vicinity of the brain, as a non-invasive solution to the drug delivery system in consideration of clinical practice. In fact, there have been reports of animal experiments showing that many types of peptides are successfully delivered to the central nervous system via an olfactory bulb or a cerebral fluid (for example, Non-Patent Document 12). However, practical use of a peptide that exhibits a central action by way of intranasal administration has yet to be realized. One major reason for this is that the characteristics of the nasal mucosa have not been considered in the development of drug delivery systems (DDS).

The nasal mucosa is covered with an olfactory epithelium and a respiratory epithelium, and the human nasal mucosa consists of an olfactory epithelium approximately by 3% and a respiratory epithelium approximately by 97% (Non-Patent Document 13). Accordingly, delivering a peptide to the human central nervous system through the respiratory epithelium, rather than through the olfactory epithelium, would be an advantageous way of intranasal administration.

The following three routes are regarded as major delivery routes for an intranasally-administered drug to the central nervous system.
(1) A route in which a drug migrates to blood at nasal mucosa, and is delivered to the central nervous system by penetrating the BBB
(2) A route in which a drug migrates to an olfactory bulb at an olfactory epithelium, or disperses in a cerebral fluid through intracellular spaces at an olfactory epithelium, and is delivered to the central nervous system
(3) A route in which a drug is delivered to the central nervous system through a respiratory epithelium and trigeminal nerves

From the viewpoint of application of drugs to clinical practice, the route (3), taking advantage of human nasal mucosa characteristics as mentioned above, may be the most appropriate way to deliver a peptide to the central nervous system. However, it is reported that the lamina propria, a lower layer of the respiratory epithelium, has a high degree of vascular permeability due to an extremely large amount of blood capillaries existing therein, and a peptide passing through the intercellular spaces in the respiratory epithelium is absorbed in the whole body. For example, a nasal formulation of calcitonin is clinically used as a therapeutic drug for osteoporosis. The nasal formulation is designed such that calcitonin is absorbed in the whole body through the nasal mucosa by way of intranasal administration (Non-Patent Document 14).

Accordingly, in order to deliver a peptide to the central nervous system in an efficient manner, suppressing the penetration of a peptide through the intracellular spaces is an important issue. From this point of view, a nose-to-brain system that exhibits an action on the central nervous system has been proposed in which a neuropeptide derivative, obtained by adding a cell-penetration accelerating sequence and an endosomal-escape accelerating sequence to a neuropeptide, is delivered to an action site such as the hippocampus or the hypothalamus by way of intranasal administration (Patent Document 1).

### [Prior Art Documents]

### [Patent Document]

Patent Document 1: International Publication No. 2016/035820

### [Non-Patent Documents]

Non-Patent Document 1: Neuroscience Research 64 (2009) 67-74
Non-Patent Document 2: Journal of Neuroscience Research 92 (2014) 446-454
Non-Patent Document 3: Behavioural Brain Research 204 (2009) 235-240
Non-Patent Document 4: Neuroscience 212 (2012) 140-148
Non-Patent Document 5: Life Sciences 93 (2013) 889-896
Non-Patent Document 6: Neuroscience Letters 550 (2013) 104-108
Non-Patent Document 7: Behavioural Brain Research 243 (2013) 153-157
Non-Patent Document 8: Neuropeptides 49 (2015) 7-14
Non-Patent Document 9: Neuroscience 294 (2015) 156-165
Non-Patent Document 10: Neuroscience Research 61 (2008) 113-119
Non-Patent Document 11: NeuroRx 2 (2005) 3-14
Non-Patent Document 12: European Journal of Pharmaceutical Sciences 40 (2010) 385-403
Non-Patent Document 13: Toxicologic Pathology 19 (1991) 321-336
Non-Patent Document 14: European Journal of Pharmaceutics and Biopharmaceutics 88 (2014) 8-27
Non-Patent Document 15: International Journal of Pharmaceutics 515 (2016) 37-45
Non-Patent Document 16: Nature 422 (2003) 37-44
Non-Patent Document 17: Neuroscientist 20 (2014) 71-81

### [Summary of the Invention]

### [Problem to be Solved by the Invention]

The neuropeptide derivative obtained by adding a cell-penetration accelerating sequence and an endosomal-escape accelerating sequence to a neuropeptide described in Patent Document 1 is delivered to the central nervous system by intranasal administration and exhibits a central action. However, the neuropeptide derivative described in Patent Document 1 tends to be highly lipophilic and poorly soluble with respect to an aqueous medium. The reason for this may be that the neuropeptide derivative has a low degree of water solubility because a sequence containing highly hydrophobic amino acid residues, such as phenylalanine, is used for promoting the endosomal escape of the neuropeptide derivative. Therefore, the neuropeptide derivative is dissolved in 16% dimethylsulfoxide (DMSO) in the tests performed in the Examples described in Patent Document 1. However, since DMSO as an organic solvent is reported to have cellular cytotoxicity or stimulating properties for eyes or skins, the toxic nature of DMSO is an issue of concern in view of clinical application. Therefore, in order to productize the neuropeptide derivative as a clinical formulation, it is necessary to improve the solubility thereof with respect to an aqueous medium. As a means for improving the solubility of a drug that is poorly soluble with respect to an aqueous medium, additives such as a surfactant or an inclusion compound are generally used. However, there have been reports of cellular cytotoxicity caused by these additives (Patent Document 15).

Further, the neuropeptide derivative described in Patent Document 1 has room to improve in terms of drug retention in the central nervous system, continuity in drug efficacy, and reduction in medicinally effective amount.

The present invention aims to provide a glycosylated neuropeptide derivative, a pharmaceutical composition, an intranasal/nasal drop formulation and a use of the glycosylated neuropeptide derivative, which exhibit excellent effects in terms of drug retention in the central nervous system, continuity in drug efficacy, and reduction in medicinally effective amount.

### [Means for Solving the Problem]

The means for solving the problem includes the following embodiments.
<1> A glycosylated neuropeptide derivative, comprising:
   a neuropeptide sequence;
   a central-nerve migration accelerating sequence that includes a cell-penetration accelerating sequence and an endosomal-escape accelerating sequence; and
   a glycosylation molecule that includes a sugar chain.
<2> The glycosylated neuropeptide derivative according to <1>, further comprising a spacer sequence that is disposed between the neuropeptide sequence and the central-nerve migration accelerating sequence, wherein the glycosylation molecule is bound to the spacer sequence.
<3> The glycosylated neuropeptide derivative according to <2>, wherein the spacer sequence includes a lysine residue, and the glycosylation molecule is bound to the lysine residue.
<4> The glycosylated neuropeptide derivative according to any one of <1> to <3>, wherein the glycosylation molecule further includes a linker.
<5> The glycosylated neuropeptide derivative according to <4>, wherein the linker includes an alkylene group with a carbon number of from 3 to 15.
<6> The glycosylated neuropeptide derivative according to any one of <1> to <5>, wherein the glycosylation molecule is bound to a C-terminus side or an N-terminus side of the neuropeptide sequence.
<7> The glycosylated neuropeptide derivative according to any one of <1> to <6>, wherein a number of monosaccharide residues per sugar chain is from 5 to 20.
<8> The glycosylated neuropeptide derivative according to any one of <1> to <7>, wherein a number of amino acid residues of the neuropeptide sequence is 200 or less.
<9> The glycosylated neuropeptide derivative according to any one of <1> to <8>, wherein the cell-penetration accelerating sequence is cationic.
<10> The glycosylated neuropeptide derivative according to any one of <1> to <9>, wherein half or more of a total number of amino acid residues of the cell-penetration accelerating sequence are basic amino acid residues.
<11> The glycosylated neuropeptide derivative according to any one of <1> to <10>, wherein the endosomal-escape accelerating sequence is an amino acid sequence selected from the group consisting of FFLIPKG, LILIG, FFG, FFFFG and FFFFFFG.
<12> A pharmaceutical composition, comprising the glycosylated neuropeptide derivative according to any one of <1> to <11> as an active ingredient.
<13> The pharmaceutical composition according to <12>, used for therapy of a neuropsychiatric disorder or a neurodegenerative disorder.
<14> The pharmaceutical composition according to <12>, used for therapy of depression or dementia.
<15> An intranasal/nasal drop formulation, comprising the glycosylated neuropeptide derivative according to any one of <1> to <11> as an active ingredient.
<16> The intranasal/nasal drop formulation according to <15>, used for therapy of a neuropsychiatric disorder or a neurodegenerative disorder.
<17> The intranasal/nasal drop formulation according to <15>, used for therapy of depression or dementia.
<18> Use of the glycosylated neuropeptide derivative according to any one of <1> to <11> for intranasal/nasal drop administration.

### [Effect of the Invention]

According to the present invention, it is possible to provide a glycosylated neuropeptide derivative, a pharmaceutical composition, an intranasal/nasal drop formulation and a use of the glycosylated neuropeptide derivative, which exhibit excellent effects in terms of drug retention in the central nervous system, continuity in drug efficacy, and reduction in medicinally effective amount.

### [Brief Explanation of the Drawings]

FIG. 1 is a graph showing the solubility of various GLP-2 derivatives in Example 1.
FIG. 2 is a graph showing the anti-depressant-like effects after intranasal administration of various GLP-2 derivatives in Example 2.
FIG. 3 is a graph showing the effect of PBS on anti-depressant-like effects of glycosylated GLP-2 derivatives (C-terminus 11-sugar) in Example 3.
FIG. 4 is an image obtained by optical imaging device showing the distribution of intranasally-administered PAS-CPP-GLP-2 derivative (no sugar) and PAS-CPP-GLP-2 derivative (C-terminus 11-sugar) in Example 4.
FIG. 5 is a graph showing the amount of PAS-CPP-GLP-2 derivative (no sugar) and PAS-CPP-GLP-2 derivative (C-terminus 11-sugar) delivered to the brain, quantified by ELISA in Example 5.
FIG. 6A is an image showing the distribution in the brain of PAS-CPP-GLP-2 derivative (no sugar) and PAS-CPP-GLP-2 derivative (C-terminus 11-sugar) obtained by immunostaining, 5 minutes after intranasal administration in Example 6.
FIG. 6B is an image showing the distribution in the brain of PAS-CPP-GLP-2 derivative (no sugar) and PAS-CPP-GLP-2 derivative (C-terminus 11-sugar) obtained by immunostaining, 20 minutes after intranasal administration in Example 6.
FIG. 7A is an image showing the distribution in the brain qualitatively and quantitatively, 5 minutes after intranasal administration in Example 7.
FIG. 7B is an image showing the distribution in the brain qualitatively and quantitatively, 20 minutes after intranasal administration in Example 7.
FIG. 7C is an image showing the distribution in the brain qualitatively and quantitatively, 60 minutes after intranasal administration in Example 7.
FIG. 8 is an image showing the localization in the trigeminal nerve of PAS-CPP-GLP-2 derivative (C-terminus 11-sugar), 5 minutes after intranasal administration in Example 8.
FIG. 9 is an image confirming the delivery to the trigeminal lemniscus of intranasally-administered glycosylated GLP-2 derivative in Example 9.
FIG. 10 is a graph showing the anti-depressant-like effects of PAS-CPP-GLP-2 derivative (C-terminus 11-sugar) and PAS-CPP-GLP-2 derivative (no sugar) after intranasal administration in Example 10.
FIG. 11 is a graph showing the anti-depressant-like effects of PAS-CPP-GLP-2 derivative (C-terminus 11-sugar) and PAS-CPP-GLP-2 derivative (no sugar) after intranasal administration in Example 11.
FIG. 12 is a graph showing the engagement of micropinocytosis of PAS-CPP-GLP-2 derivative (C-terminus 11-sugar) in the cellular uptake mechanism into neuron cells Neuro A2 in Example 12.
FIG. 13A is a graph showing the effect of improvement in learning and memory functions of glycosylated PAS-CPP-GLP-1 derivative (C-terminus 11-sugar) and PAS-CPP-GLP-1 derivative (no sugar) after intranasal administration in Example 13.
FIG. 13B is a graph showing the effect of improvement in learning and memory functions of PAS-CPP-GLP-1 derivative (no sugar) after intranasal administration or intraventricular administration.
FIG. 14A is an image showing the ability of PAS-CPP-GLP-1 derivative (no sugar) and glycosylated PAS-CPP-GLP-1 derivative (N-terminus 11-sugar) to migrate to the brain studied by in vivo imager in Example 14.
FIG. 14B is a graph showing the quantification results of the amount of PAS-CPP-GLP-1 derivative (no sugar) and glycosylated PAS-CPP-GLP-1 derivative (N-terminus 11-sugar) to migrate to the brain.
FIG. 14C is a graph showing the effect of PAS-CPP-GLP-1 derivative (no sugar) and glycosylated PAS-CPP-GLP-1 derivative (N-terminus 11-sugar) to improve learning and memory functions after intranasal administration.
FIG. 14D is a graph showing the effect of PAS-CPP-GLP-1 derivative (no sugar) and glycosylated PAS-CPP-GLP-1 derivative (N-terminus 11-sugar) to improve learning and memory functions 60 minutes after intranasal administration.
FIG. 14E is a graph showing the effect of PAS-CPP-GLP-1 derivative (no sugar) and glycosylated PAS-CPP-GLP-1 derivative (N-terminus 11-sugar) to improve learning and memory functions 5 minutes after intranasal administration.
FIG. 14F is a graph showing the anti-depressant-like effect of PAS-CPP-GLP-2 derivative (no sugar) after intranasal administration.
FIG. 14G is a graph showing the anti-depressant-like effect of glycosylated PAS-CPP-GLP-2 derivative (N-terminus 11-sugar) after intranasal administration.
FIG. 15A is a graph showing the amount of uptake in neuron cells of various glycosylated PAS-CPP-GLP-2 derivatives in Example 15.
FIG. 15B is a graph showing the anti-depressant-like effect of various glycosylated PAS-CPP-GLP-2 derivatives in Example 15.
FIG. 15C is a graph showing the neural activity at acting sites of various glycosylated PAS-CPP-GLP-2 derivatives in Example 15.
FIG. 15D is a graph showing the quantification results of the amount of various glycosylated PAS-CPP-GLP-2 derivatives to migrate to the brain.
FIG. 16 is a graph showing the usefulness of PAS-CPP in PAS-CPP-GLP-2 derivative.
FIG. 17 is a graph showing the effect of glycosylated PAS-CPP-GLP-1 derivative to suppress amyloid β-induced neurodegeneration in Example 16.
FIG. 18 is a graph showing the effect of glycosylated PAS-CPP-GLP-1 derivative to suppress amyloid β-induced neurodegeneration in Example 16.
FIG. 19 is a graph showing the effect of glycosylated PAS-CPP-GLP-1 derivative to suppress amyloid β-induced neurodegeneration in Example 16.

### [Embodiments for Implementing the Invention]

In the following, embodiments of the invention are explained. The explanation and the embodiments described herein are intended to exemplify the invention, and do not limit the scope of the invention.

Numerical ranges indicated as "from A to B" described herein include A and B as a minimum value and a maximum value, respectively. The amino acid sequence is described such that the left side is N-terminus and the right side is C-terminus. The amino acid residue may be indicated by a one-letter abbreviation known in the art (for example, G for glycine residue).

The term "treatment" as described herein refers not only to an activity or an effect that quenches or alleviates a symptom, but also to an activity or an effect that suppresses aggravation of the symptom. The term "anti-depressant activity" or "anti-depressant effect" refers not only to an activity or an effect that quenches a symptom of depression, but also to an activity or an effect that suppresses aggravation of the symptom. The term "learning disorder-alleviating activity" or "learning disorder-alleviating effect" refers not only to an activity or an effect that quenches a symptom of a learning disorder, but also to an activity or an effect that suppresses aggravation of the symptom.

### <Glycosylated neuropeptide derivative>

The glycosylated neuropeptide derivative of the present invention has a neuropeptide sequence; a central-nerve migration accelerating sequence, which includes a cell-penetration accelerating sequence (hereinafter, also referred to as a cell penetrating peptide or CPP) and an endosomal-escape accelerating sequence (hereinafter, also referred to as a penetration accelerating sequence or PAS); and a glycosylation molecule that includes a sugar chain.

As a solution for the problem of being poorly soluble with respect to an aqueous medium of neuropeptide derivatives, the inventor has produced a neuropeptide derivative to which a sugar chain is added (hereinafter, also referred to as a glycosylated neuropeptide derivative), without using an additive such as a surfactant or an inclusion compound that may cause damage to cells. Generally, neuropeptide derivatives have a problem of a trade-off between water solubility and membrane permeability. Therefore, there has been anxiety that while addition of a sugar chain to a neuropeptide derivative may improve the water solubility, it may increase the amount of the neuropeptide derivative required to exhibit pharmaceutical effects, or may cancel the pharmaceutical effects of the neuropeptide derivative.

However, the addition of a sugar chain to a neuropeptide derivative has resulted in surprising findings. Specifically, when a glycosylated neuropeptide derivative is administered in an intranasal manner, it is delivered to the central nervous system and remains in such an efficient manner that could not have been expected based on common knowledge, as compared with a neuropeptide derivative to which a sugar chain is not added. Further, the addition of a sugar chain to a neuropeptide derivative proves to increase the continuity of pharmaceutical effects and reduce the medicinally effective amounts. Further, the addition of a sugar chain to a neuropeptide derivative proves to cause similar effects not only on a neuropeptide derivative with poor water solubility but also on a neuropeptide derivative that is soluble in water.

A possible pathway for a glycosylated neuropeptide derivative to reach the central nervous system after being intranasally administered may be a pathway from the trigeminal nerve and trigeminal ganglion in the nasal cavity to the central nervous system such as the hippocampus or the hypothalamus, via the principal sensory nucleus of trigeminal nerve (Pr5) that exists at the pons in the brainstem.

A possible pathway from the principal sensory nucleus of trigeminal nerve to the central nervous system may be a trigeminal lemniscus. The trigeminal lemniscus may be referred to as the trigeminothalamic tract. In the present disclosure, the trigeminal lemniscus is a concept that includes the trigeminothalamic tract.

The glycosylated neuropeptide derivative of the present invention has a cell-penetration accelerating sequence and an endosomal-escape accelerating sequence. As shown in the Reference Example, while a derivative in which both of a cell-penetration accelerating sequence and an endosomal-escape accelerating sequence are added to a neuropeptide sequence exhibits an anti-depressant activity as a central activity, a derivative in which either one of a cell-penetration accelerating sequence or an endosomal-escape accelerating sequence is added to a neuropeptide sequence does not exhibit an anti-depressant activity. In other words, the achievement of excellent central activity by the glycosylated neuropeptide derivative of the present invention is attributable to the addition of both of a cell-penetration accelerating sequence and an endosomal-escape accelerating sequence.

The intended purpose of the glycosylated neuropeptide derivative is not particularly limited, as long as it makes use of a pharmacological activity expressed by the glycosylated neuropeptide derivative upon action on the central nervous system. Examples of the pharmacological activity include an anti-depressant activity, a learning disorder-alleviating activity, an anti-anxiety activity, a feeding suppression activity, a cognitive disorder-alleviating activity, a blood-pressure-lowering activity, an analgesic activity, a sleep-inducing activity, and an anti-epileptic activity. Therefore, the glycosylated neuropeptide derivative of the present invention is suitably used as a drug for the treatment of neuropsychiatric disorders or neurodegenerative disorders, such as an anti-depressant agent, a learning disorder-alleviating agent, an anti-anxiety agent, a feeding suppression agent, a cognitive disorder-alleviating agent, a blood-pressure-lowering agent, an analgesic agent, a sleep-inducing agent, and an anti-epileptic agent.

The number of amino acid residues included in the glycosylated neuropeptide derivative is not particularly limited. As shown in the Examples, it is confirmed that the glycosylated neuropeptide derivative is taken into nerve cells by way of macropinocytosis. The macropinocytosis is a system that causes cellular uptake by means of reconstruction of an actin skeleton and formation of a ruffling structure of fluid plasma membranes, and produces endosomal vesicles with a size of greater than 1 µm. Accordingly, it is expected that a glycosylated neuropeptide derivative with a large molecular size may be taken into a cell (Non-Patent Document 16).

For example, the total number of amino acid residues included in the glycosylated neuropeptide derivative may be determined depending on the total number of the neuropeptide sequence, cell-penetration accelerating sequence, endosomal-escape accelerating sequence and a spacer sequence. For example, the total number of amino acid residues included in the glycosylated neuropeptide derivative may be 250 or less, 200 or less, or 150 or less. The total number of amino acid residues included in the glycosylated neuropeptide derivative may be 10 or more, 20 or more, or 30 or more.

Each of the amino acid residues that constitutes the glycosylated neuropeptide derivative may be either L-form or D-form, as long as the effect of the invention is achieved. The method for producing the glycosylated neuropeptide derivative is not particularly restricted, and may be a method of extracting from a living body or a natural substance, or may be prepared by a genetic engineering process or an organic synthetic process.

### (Neuropeptide sequence)

The neuropeptide sequence in the glycosylated neuropeptide sequence derivative is not particularly limited, as long as it is derived from a peptide that exhibits pharmaceutical effects by acting on the central nervous system. The method for adding a cell-penetration accelerating sequence, an endosomal-escape accelerating sequence and a sugar chain to a neuropeptide sequence is not particularly limited, and may be performed by a known method.

The number of amino acid residues included in the neuropeptide sequence in the glycosylated neuropeptide sequence derivative is not particularly limited, as long as the glycosylated neuropeptide derivative can be taken into cells by way of macropinocytosis, and may be determined in view of the nature of macropinocytosis. The total number of amino acid residues included in the neuropeptide sequence may be from 5 to 200, from 5 to 170, from 9 to 120, from 9 to 70, or from 9 to 60. The total number of amino acid residues included in the neuropeptide sequence may be from 5 or more, 10 or more, or 15 or more. The total number of amino acid residues included in the neuropeptide sequence may be 200 or less, 170 or less, 120 or less, 70 or less, 60 or less, or 51 or less.

In an embodiment, the neuropeptide sequence is an amino acid sequence derived from a neuropeptide having a centrally-acting activity.

Specific examples of the neuropeptide include GLP-1 (23 amino acid residues), GLP-2 (37 amino acid residues), enkephalin (5 amino acid residues), pasireotide (5 amino acid residues), oxytocin enkephalin (5 amino acid residues), ocretide (7 amino acid residues), lanreotide (7 amino acid residues), oxytocin (9 amino acid residues), somatostatin-14 (14 amino acid residues), dynorphin (17 amino acid residues), somatostatin-28 (28 amino acid residues), ghrelin (28 amino acid residues), orexin B (28 amino acid residues), galanin (30 amino acid residues), β-endorphin (31 amino acid residues), orexin A (33 amino acid residues), neuropeptide Y (36 amino acid residues), insulin (51 amino acid residues), galanin-like peptide (60 amino acid residues), insulin-like growth factor-1 (70 amino acid residues), nerve growth factor (118 amino acid residues), leptin (166 amino acid residues), dynorphin (17 amino acid residues), ghrelin (28 amino acid residues), orexin B (28 amino acid residues), galanin (30 amino acid residues), β-endorphin (31 amino acid residues), orexin A (33 amino acid residues), neuropeptide Y (36 amino acid residues), insulin (51 amino acid residues), galanin-like peptide (60 amino acid residues), insulin-like growth factor-1 (70 amino acid residues), nerve growth factor (118 amino acid residues) and leptin (166 amino acid residues).

In an embodiment of the neuropeptide derivative, the neuropeptide sequence is an amino acid sequence derived from a peptide that is any of the following (a1) to (a2) or (b).
(a1) a peptide formed of an amino acid sequence represented by HADGSFSDEMNTILDNLAARDFINWLIQTKITD (GLP-2, SEQ ID NO: 1)
(a2) a peptide formed of an amino acid sequence represented by HAEGTFTSDVSSYLEGQAAKEFIAWLVKGR-NH₂ (GLP-1: active-type 7-36 amide, SEQ ID NO: 2)
(b) a peptide formed of an amino acid sequence represented by (a1) or (a2) in which one or several amino acid residues are deleted, replaced or added, the peptide having a centrally-acting activity.

Among these peptides, GLP-2 is known to exert an anti-depressant activity and a blood-pressure-lowering activity, and GLP-1 is known to exert a learning disorder-alleviating activity. Therefore, these peptides are useful as a neuropeptide sequence.

When a peptide is formed from a combination of an amino acid sequence of a specific peptide with other amino acid sequences, the term "amino acid sequence derived from a specific peptide" refers to a portion of the peptide corresponding to an amino acid sequence of the specific peptide.

When the neuropeptide sequence is derived from "(b) a peptide formed of an amino acid sequence represented by (a1) or (a2) in which one or several amino acid residues are deleted, replaced or added", the number of the amino acid residues to be deleted, replaced or added is not particularly limited, as long as the neuropeptide sequence can exert the effect of the invention. For example, the number of the amino acid residues to be deleted, replaced or added may be from 1 to 10, preferably from 1 to 5, more preferably from 1 to 3.

### (Cell-penetration accelerating sequence)

The cell-penetration accelerating sequence in the glycosylated neuropeptide derivative is an amino acid sequence derived from a cell-penetrating peptide, i.e., a peptide that exerts an activity to penetrate a cell membrane.

Examples of the cell-penetrating peptide include oligoarginine (Rn, n is the number of arginine residues ranging from 6 to 12), oligolysine (Kn, n is the number of lysine residues ranging from 6 to 12), penetratin (RQIKIWFQNRRMKWKK, 16 amino acid residues, SEQ ID NO: 3), TAT (GRKKRRQRRR, 10 amino acid residues, SEQ ID NO: 4), mini penetratin (RRMKWKK, 7 amino acid residues, SEQ ID NO: 5), R9FC (RRRRRRRRRFFC, 12 amino acid residues, SEQ ID NO: 6), AIP6 (RLRWR, 5 amino acid residues, SEQ ID NO: 7), DPV3 (RKKRRRESRKKRRRES, 16 amino acid residues, SEQ ID NO: 8), DPV6 (GRPRESGKKRKRKRLKP, 17 amino acid residues, SEQ ID NO: 9), Pep-1 (KETWWETWWTEWSQPKKKRKV, 21 amino acid residues, SEQ ID NO: 10), MPG (GLAFLGFLGAAGSTMGAWSQPKKKRKV, 27 amino acid residues, SEQ ID NO: 11), Transportan (GWTLNSAGYLLGKINLKALAALAKKIL, 27 amino acid residues, SEQ ID NO: 12), MAP (KLALKALKALKAALKLA, 17 amino acid residues, SEQ ID NO: 13), W/R (RRWWRRWRR, 9 amino acid residues, SEQ ID NO: 14), CADY (GLWRALWRLLRSLWRLLWRA, 20 amino acid residues, SEQ ID NO: 15), EB-1 (LIRLWSHLIHIWFQNRRLKWKK, 22 amino acid residues, SEQ ID NO: 16), HRSV (RRIPNRRPRR, 10 amino acid residues, SEQ ID NO: 17), PTD-5 (RRQRRRTSKLMKR, 13 amino acid residues, SEQ ID NO: 18), TAT47-57 (YGRKKRRQRRR, 11 amino acid residues, SEQ ID NO: 19), TP2 (PLIYLRLLRGQF, 12 amino acid residues, SEQ ID NO: 20), TP10 (AGYLLGKINLHALAALAKKIL, 21 amino acid residues, SEQ ID NO: 21), a cationic sequence that binds to heparan, a cationic sequence that binds to RNA, and a cationic sequence that binds to DNA.

The cell-penetrating peptide that constitutes the cell-penetration accelerating sequence is preferably a cell-penetrating peptide that is positively charged as a whole, or a cell-penetrating peptide that is cationic as a whole. For example, a cationic cell-penetrating peptide formed of an amino acid sequence that is rich in basic amino acid residues such as arginine, lysine, histidine or tryptophan (for example, an amino acid sequence in which half or more of the total amino acid residues are basic amino acid residues) is preferred. Examples of such cell-penetrating peptides include oligoarginine (Rn, n is the number of arginine residues ranging from 6 to 12), TAT derived from a Tat protein of human immunodeficiency virus 1 (HIV-1), penetratin, Pep-1, MPG, MAP, CADY, EB-1 and Transportan.

It is thought that a cell-penetrating peptide formed of an amino acid sequence that is rich in basic amino acid residues induces macropinocytosis as a form of endocytosis, which is a process of a cell to take in an extracellular substance. As such, it is thought that the glycosylated neuropeptide derivative is introduced into a cell in a more efficient manner.

The number of amino acid residues of the cell-penetration accelerating sequence is preferably from 5 to 27. Further, the cell-penetration accelerating sequence is preferably a peptide in which half or more of the total amino acid residues are basic amino acid residues; more preferably a peptide that includes arginine residues as the basic amino acid residues; further preferably an oligoarginine formed of 6 to 12 arginine residues; yet further preferably an oligoarginine formed of 7 to 9 arginine residues; yet further preferably an oligoarginine formed of 8 arginine residues.

### (Endosomal-escape accelerating sequence)

It is thought that the endosomal-escape accelerating sequence shortens the time for the glycosylated neuropeptide derivative, which has been introduced into a cell, to remain in an endosome, and allows the glycosylated neuropeptide derivative to escape from an endosome in a shorter period. As a result, it is thought that the delivery of the glycosylated neuropeptide derivative to the central nervous system and the distribution of the glycosylated neuropeptide derivative in the central nervous system can be achieved in a shortened period.

The structure of the endosomal-escape accelerating sequence is not particularly limited, and examples thereof include amino acid sequences having an activity to promote endosomal escape such as FFLIPKG (SEQ ID NO: 22), LILIG (SEQ ID NO: 23), FFG, FFFFG (SEQ ID NO: 24) and FFFFFFG (SEQ ID NO: 25).

The position of the cell-penetration accelerating sequence and the endosomal-escape accelerating sequence in the glycosylated neuropeptide derivative is not particularly limited. For example, either one of the cell-penetration accelerating sequence or the endosomal-escape accelerating sequence may be disposed at a position closer to the neuropeptide sequence.

From the viewpoint of achieving the effect of the present invention in a more efficient manner, it is preferred that the cell-penetration accelerating sequence is disposed at a position closer to the neuropeptide sequence; and it is more preferred that the cell-penetration accelerating sequence is disposed at a position closer to the neuropeptide sequence and the endosomal-escape accelerating sequence is disposed at a N-terminus side or a C-terminus side of the cell-penetration accelerating sequence.

### (Glycosylation molecule)

The type of the sugar chain in the glycosylation molecule is not particularly limited. Specific examples of the sugar chain include N-linked sugar chains of high-mannose type, composite type or hybrid type (combination of high-mannose type and composite type), O-linked sugar chains, and proteoglycans such as mucin-type proteoglycan, heparan sulfate, chondroitin sulfate, keratan sulfate, hyaluronic acid and dermatan sulfate. Among these sugar chains, N-linked sugar chains are preferred, and composite-type N-linked sugar chains are more preferred.

The configuration of the sugar chain is not particularly limited, and may be a biantennary structure or other configurations (such as a branched configuration). Examples of the monosaccharide that constitutes a sugar chain include glucose, mannose, galactose, fructose, N-acetylglucosamine, N-acetylgalactosamine, N-acetylmannosamine, fucose, sialic acid, N-acetylneuramic acid, N-glycolylneuramic acid, deaminoneuramic acid, glucuronic acid, iduronic acid, galacturonic acid, xylose, ribose and deoxyribose. The monosaccharide that constitutes the sugar chain may be either D-form or L-form. The monosaccharide that constitutes the sugar chain may be either an α-anomer or a β-anomer.

The number of the glycosylation molecule included in the glycosylated neuropeptide derivative may be 1 or 2 or more. The number of the sugar chain included in a single glycosylation molecule may be 1 or 2 or more.

The number of the sugar chain in the glycosylated neuropeptide derivative may be 1 or 2 or more.

In the present disclosure, the number of the sugar chain in the glycosylated neuropeptide derivative is counted based on a basal portion of the sugar chain. Specifically, a group of monosaccharide residues stemming from a single basal portion is regarded as a single sugar chain.

The glycosylation molecule may be composed only of a sugar chain, or may be composed of a sugar chain and a portion other than the sugar chain. Namely, the sugar chain may bind to an amino acid residue that constitutes the glycosylated neuropeptide derivative in a direct manner or an indirect manner. In the present disclosure, each of a state in which a sugar chain directly binds to an amino acid residue that constitutes the glycosylated neuropeptide derivative, or a state in which a sugar chain indirectly binds to an amino acid residue that constitutes the glycosylated neuropeptide derivative, is regarded as a state in which the glycosylation molecule binds to an amino acid residue that constitutes the glycosylated neuropeptide.

Examples of a state in which a sugar chain indirectly binds to an amino acid residue that constitutes the glycosylated neuropeptide derivative include a state in which a sugar chain binds to an amino acid residue that constitutes the glycosylated neuropeptide derivative via an amino acid residue such as a cysteine residue or an asparagine residue, or via a hydrocarbon group.

It is preferred that the glycosylation molecule binds to a portion such that the functions of the cell-penetration accelerating sequence or the endosomal-escape accelerating sequence are favorably maintained, and it is more preferred that the glycosylation molecule binds to the neuropeptide sequence.

When the glycosylation molecule binds to the neuropeptide sequence, the binding site for the glycosylation molecule is not particularly limited, as long as the stability or the activity of the neuropeptide is not lowered. Specifically, the binding site for the glycosylation molecule may be any of C-terminus side, N-terminus side, or other portions of the neuropeptide.

It is possible to substitute a portion of the neuropeptide sequence with an amino acid residue to which a glycosylation molecule is likely to bind, such as a cysteine residue or an asparagine residue, as long as the physiological activity of the neuropeptide is not affected. Alternatively, it is possible to introduce an amino acid residue at a terminus of the neuropeptide and allow the glycosylation molecule to bind thereto.

From the viewpoint of avoid the risk of affecting the function of the cell-penetration accelerating sequence or the endosomal-escape accelerating sequence, the glycosylation molecule is preferably bound at a position at which an appropriate distance between the glycosylation molecule and the cell-penetration accelerating sequence or the endosomal-escape accelerating sequence can be secured.

The binding site for the glycosylation molecule to the neuropeptide is not particularly limited, as long as the glycosylation molecule does not affect the function of the cell-penetration accelerating sequence or the endosomal-escape accelerating sequence, and does not affect the physiological activity of the neuropeptide. For example, when the cell-penetration accelerating sequence or the endosomal-escape accelerating sequence is added at the N-terminus side of the neuropeptide sequence, the glycosylation molecule is preferably bound at the C-terminus side of the neuropeptide sequence. When the cell-penetration accelerating sequence or the endosomal-escape accelerating sequence is added at the C-terminus side of the neuropeptide sequence, the glycosylation molecule is preferably bound at the N-terminus side of the neuropeptide sequence.

From the viewpoint of adjusting the distance between the glycosylation molecule and the central-nerve migration accelerating sequence, the glycosylation molecule preferably includes a linker.

The type of the linker is not particularly limited, and examples thereof include an alkylene group and a polyethylene glycol (PEG) group.

When the linker is an alkylene group, the carbon number thereof is preferably from 3 to 15, more preferably from 4 to 12, further preferably from 5 to 10, from the viewpoint of securing an appropriate distance between the glycosylation molecule and the central-nerve migration accelerating sequence.

The number of monosaccharide residues per sugar chain included in the glycosylation molecule is not particularly limited, and may be in a range of from 5 to 20, or from 5 to 15, for example.

The study conducted by the inventor proves that the solubility of the neuropeptide derivative with respect to an aqueous medium is improved by modifying the same with a glycosylation molecule that includes a sugar chain composed of at least a certain number of monosaccharide residues. Specifically, the number of monosaccharide residues per sugar chain is preferably 5 or more, more preferably 10 or more.

### (Spacer sequence)

The glycosylated neuropeptide derivative may have a spacer sequence that is disposed between the neuropeptide sequence and the central-nerve migration accelerating sequence. When a spacer sequence is disposed between the neuropeptide sequence and the central-nerve migration accelerating sequence, the spacer sequence may exert an effect of preventing the activity of the neuropeptide sequence from lowering or deteriorating.

When the glycosylated neuropeptide derivative has a spacer sequence, the spacer sequence may be disposed between the neuropeptide sequence and the cell-penetration accelerating sequence, or may be disposed between the neuropeptide sequence and the endosomal-escape accelerating sequence.

Generally, the cell-penetration accelerating sequence is formed of basic amino acid residues. Therefore, when the neuropeptide sequence includes acidic amino acid residues, it may be possible to prevent the cell-penetration accelerating sequence and the neuropeptide sequence from interacting with each other, and avoid the lowering or degradation of the activity of the neuropeptide sequence, by disposing a spacer sequence including neutral amino acid residues such as glycine, for example, by the number of from 1 to 10, preferably from 2 to 6.

From the viewpoint of securing a distance between the glycosylation molecule and the central-nerve migration accelerating sequence, the spacer sequence preferably includes a lysine (K) residue. Lysine is an amino acid having a 4-aminobutyl group as a side chain. It is possible to dispose an alkylene group with a carbon number of 4 between the glycosylation molecule and the spacer sequence by binding the glycosylation molecule to a terminal amino group in the 4-aminobutyl group. Therefore, it is possible to secure an appropriate distance between the glycosylation molecule and the central-nerve migration accelerating sequence.

Examples of the spacer sequence including a lysine residue include those obtained by substituting one or more neutral amino acid residues such as glycine with a lysine residue, of a spacer sequence with the number of amino acid residues of from 1 to 10, preferably from 2 to 6.

The glycosylated neuropeptide derivative may be subjected to various kinds of modification according to the intended purposes, in addition to the addition of a sugar chain. Examples of the modification include amino group modification (such as biotinylation, myristoylation, palmitoylation, acetylation and maleimidation), carboxy group modification (such as amidation and esterification), thiol group modification (such as farnesylation, geranylation, methylation and palmitoylation), hydroxy group modification (such as phosphorylation, sulfation, octanoylation, palmitoylation and palmitoleoylation, fluorescence labelling (such as FITC, FAM, rhodamine, BODIPY, NBD and MCA), pegylation, and introduciton of amino acids such as an unnatural amino acid or a D-amino acid. The modification may be performed on any of the neuropeptide sequence, cell-penetration accelerating sequence, endosomal-escape accelerating sequence or spacer sequence of the glycosylated neuropeptide derivative.

The combination of the neuropeptide sequence, cell-penetration accelerating sequence and endosomal-escape accelerating sequence as the constituents of the glycosylated peptide derivative is not particularly limited, and may be selected depending on the intended purposes.

In an embodiment, the glycosylated peptide derivative may have an endosomal-escape accelerating sequence, a cell-penetration accelerating sequence, a spacer sequence as necessary, and a neuropeptide sequence, in this order from the N-terminus side.

In an embodiment, the glycosylated peptide derivative may have an endosomal-escape accelerating sequence, a cell-penetration accelerating sequence, a spacer sequence as necessary, and a neuropeptide sequence, in this order from the C-terminus side.

In the aforementioned configurations, the endosomal-escape accelerating sequence may be selected from FFLIPKG, LILIG, FFG, FFFFG or FFFFFFG.

In the aforementioned configurations, the cell-penetration accelerating sequence may be selected from oligoarginines (for example, Rn, n=6-12).

In the aforementioned configurations, the spacer sequence may be selected from a sequence formed of a glycine residue (for example, Gn, n=2-6), a sequence formed of a glycine residue and a cysteine residue (for example, GCG) and a sequence formed of a glycine residue and a lysine residue (for example, GKG).

### <Pharmaceutical composition>

The pharmaceutical composition of the present invention includes the foregoing glycosylated neuropeptide derivative as an active ingredient. By including the glycosylated neuropeptide derivative as an active ingredient, the pharmaceutical composition can be rapidly delivered to the central nervous system and express a pharmacological effect efficiently. Therefore, for example, the pharmaceutical composition is suitable for the therapy of diseases in which daily medication at home is required. Accordingly, preferred examples of formulation of the pharmaceutical composition include intranasal/nasal drop formulations.

The neuropsychiatric disorder or neurodegenerative disorder as a therapeutic objective for the pharmaceutical composition is not particularly limited, as long as the neuropeptide sequence in the glycosylated neuropeptide derivative acts on the central nervous system and exerts therapeutic effects on the therapeutic objective.

Examples of the neuropsychiatric disorders or neurodegenerative disorders as a therapeutic objective include depression, learning disorder, anxiety, eating disorder, cognition disorder, high blood pressure, sleeping disorder, epilepsia, Alzheimer's disease, vascular dementia, and amyotrophic lateral sclerosis.

Specific examples of the pharmaceutical composition include an anti-depressant agent, a learning disorder-alleviating agent, an anti-anxiety agent, a feeding suppression agent, a cognitive disorder-alleviating agent, a blood-pressure-lowering agent, an analgesic agent, a sleep-inducing agent, and an anti-epileptic agent. The type of the neuropeptide sequence in the glycosylated neuropeptide derivative may be selected according to the therapeutic objective.

For example, a pharmaceutical composition including a glycosylated neuropeptide derivative having a neuropeptide sequence derived from GLP-2 as an active ingredient is effective as an anti-depressant agent. Further, since GLP-2 exerts a blood pressure-lowering activity, a pharmaceutical composition including a glycosylate neuropeptide having a neuropeptide sequence derived from GLP-2 is thought to be particularly effective for a patient with both depression due to strong stress and high blood pressure. A pharmaceutical composition including a glycosylated neuropeptide derivative having a neuropeptide sequence derived from GLP-1 as an active ingredient is thought to be effective as a learning disorder-alleviating agent, and is expected as a therapeutic agent for dementia.

The details and the preferred embodiments of the glycosylated neuropeptide derivative included in the pharmaceutical composition are as described above. From the viewpoint of the ability to be delivered to the central nervous system, the method for using the pharmaceutical composition is preferably intranasal/nasal administration.

The pharmaceutical composition may include a component other than the glycosylated neuropeptide derivative. Examples of the component other than the glycosylated neuropeptide derivative include a medium and a formulation additive that are used for the preparation of a pharmaceutical composition. Specific examples of the formulation additive include a diluent, a disintegrant, a binder, a lubricant, a surfactant, a buffer, a solubilizing agent, a stabilizer, a tonicity agent, a suspending agent, an emulcifier, a solvent, a thickner, a mucolytic agent, a humectant and a preservative. The dosage amount of the pharmaceutical composition may be selected according to the type of disease, the symptomatic state, weight or age of the patient, the administration form and the like.

The pharmaceutical composition of the present invention is particularly suitable as an intranasal/nasal drop formulation. Therefore, an embodiment of the present invention is a use of the present invention for intranasal/nasal administration.

### <Intranasal/nasal drop formulation>

The intranasal/nasal drop formulation of the present inveniton includes the foregoing glycosylated neuropeptide derivative as an active ingredient. By including the glycosylated neuropeptide derivative as an active ingredient, the intranasal/nasal drop formulation can be rapidly delivered to the central nervous system and efficiently exerts a pharmacological effect. Further, since the intranasal/nasal drop formulation can be administered is a less invasive manner, it is useful for the therapy of a disease that requires daily medication at home.

The intranasal/nasal drop formulation may include a component other than the glycosylated neuropeptide derivative. Examples of the component other than the glycosylated neuropeptide derivative include a medium and a formulation additive that may be used for the preparation of the pharmaceutical composition, such as those as described above.

The embodiments of the present invention include a use of the pharmaceutical composition including a glycosylated neuropeptide derivative as an active ingredient, as described above, for intranasal administration. The details and the preferred embodiments of the glycosylated neuropeptide derivative for the use are as described above.

### <Therapeutic method for neuropsychiatric disorders or neurodegenerative disorders>

The embodiments of the present invention include a therapeutic method for neuropsychiatric disorders or neurodegenerative disorders, the method including administrating, to a patient, the glycosylated neuropeptide derivative or the pharmaceutical composition as mentioned above. The details and preferred embodiments of the glycosylated neuropeptide derivative or the pharmaceutical composition in the therapeutic method are as described above.

Specific examples of the neuropsychiatric disorders or the neurodegenerative disorders to be treated by the therapeutic method include depression, learning disorder, anxiety, eating disorder, cognition disorder, high blood pressure, sleeping disorder, epilepsia, Alzheimer's disease, vascular dementia, and amyotrophic lateral sclerosis.

While the method for administrating the glycosylated neuropeptide derivative or the pharmaceutical composition to a patient is not particularly limited, it is preferably intranasal administration.

### [Examples]

The present invention is explained in further detail by referring to the following examples. The materials, amounts, rates, procedures and the like may be modified without departing from the scope of the invention. Therefore, the scope of the present invention should not be construed in a restricted manner by the examples as described below.

### <Preparation of GLP-2 derivative glycosylated at C-terminus>

A glycosylated GLP-2 derivative referred to as PAS-CPP-GLP-2 (C-terminus 11-sugar), in which an endosomal-escape accelerating sequence (PAS: FFLIPKG), a cell-penetration accelerating sequence (CPP: RRRRRRRR), a spacer sequence (GG) and an amino acid sequence derived from GLP-2 as a neuropeptide sequence are disposed in this order from the N-terminus side, was prepared by an ordinary process. The neuropeptide sequence used for the preparation was added with a molecule including a sugar chain consisting of 11 monosaccharide residues, at the C-terminus of GLP-2 via a cysteine residue. The configuration of the glycosylated GLP-2 derivative is described below.

A fluorescent-labeled PAS-CPP-GLP-2 (C-terminus 11-sugar) used in some experiments was prepared by adding a fluorescent label (FITC or ICG) to the endosomal-escape accelerating sequence.

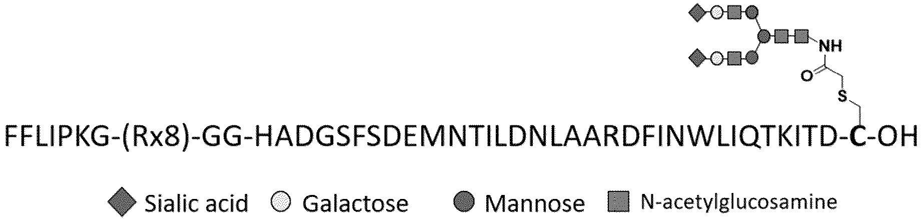

A glycosylated GLP-2 derivative referred to as PAS-CPP-GLP-2 (C-terminus 5-sugar) was prepared in the same manner as the PAS-CPP-GLP-2 derivative (C-terminus 11-sugar), except that a neuropeptide sequence added with a molecule including a sugar chain consisting of 5 monosaccharide residues was used. The configuration of the glycosylated GLP-2 derivative is described below.

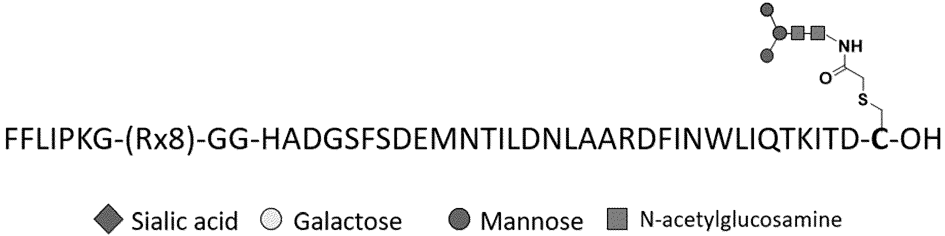

A GLP-2 derivative not subjected to glycosylation, referred to as a PAS-CPP-GLP-2 derivative (no sugar), was prepared in the same manner as the PAS-CPP-GLP-2 derivative (C-terminus 11-sugar), except that a neuropeptide sequence added with a molecule not including a sugar chain at the C-terminus of GLP-2 via a cysteine residue was used. The configuration of the GLP-2 derivative is described below.

A fluorescent-labeled PAS-CPP-GLP-2 derivative (no sugar) used in some experiments was prepared by adding a fluorescent label (FITC or ICG) to the endosomal-escape accelerating sequence.

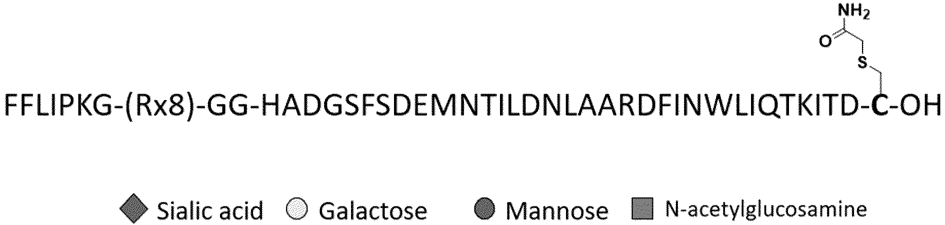

### <Example 1: evaluation on solubility of GLP-2 derivative with respect to aqueous medium>

Samples were prepared by dispensing a Milli-Q aqueous solution of PAS-CPP-GLP-2 (no sugar), PAS-CPP-GLP-2 (C-terminus 5-sugar) or PAS-CPP-GLP-2 (C-terminus 11-sugar) to a microtube by an amount of 5 nmol/tube, 30 nmol/tube, 60 nmol/tube, 120 nmol/tube or 200 nmol/tube, and freeze-drying the same. The freeze-dried samples were added with 200 µm of PBS (Dulbecco's Phosphate Buffered Saline; Sigma-Aldrich, herein after the same), subjected to ultrasonic treatment, and allowed to stand overnight. Thereafter, the concentration of the peptide was measured. Specifically, a supernatant obtained by centrifuging the sample suspension was subjected to the measurement of OD (280 nm) with a spectrometer (NanoDrop^{™} 2000c spectrometer; Thermo Fisher Scientific K.K.)

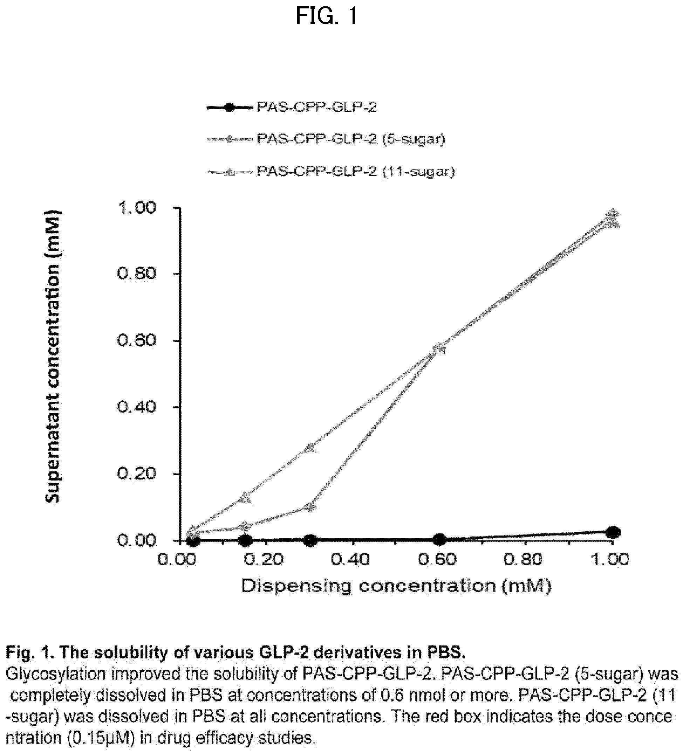

As shown in FIG. 1, the PAS-CPP-GLP-2 derivative (no sugar) was not dissolved in PBS at all. Each of the glycosylated GLP-2 derivatives (C-terminus 5-sugar or C-terminus 11-sugar) exhibited an improved solubility with respect to PBS. In particular, the solubility of the GLP-2 derivative (C-terminus 11-sugar) was linearly improved.

### <Example 2: Evaluation on influence of glycosylation on pharmaceutical effects of GLP-2 derivative>

A forced swim test (FST) was performed on mice in order to evaluate the influence of glycosylation on anti-depressant-like effects of the GLP-2 derivatives (C-terminus 11-sugar, C-terminus 5-sugar).

### (Preparation and administration of administration solution)

The following test was performed to evaluate whether or not the pharmaceutical effects are maintained by glycosylation.

Administration solutions were prepared by completely dissolving the PAS-CPP-GLP-2 derivative (no sugar) or the PAS-CPP-GLP-2 derivatives (5-sugar or 11-sugar) in DMSO and adding PBS such that the final concentration of DMSO was 16% by mass. The concentration of each of the GLP-2 derivatives was adjusted to 0.6 nmol/4 µL.

After anesthetizing mice with isoflurane using an all-in-one small animal anesthetizer (MK-AT210D, Muromachi Kikai Co., Ltd., hereinafter the same), the solution was intranasally administered to the mice. Specifically, the intranasal administration was performed by contacting the tip of the anesthetizer to a nasal cavity in a horizontal manner such that the solution was inhaled by spontaneous respiration, by the amount of 2 µL per nose cavity, 4 µL in total (0.6 nmol/mouse). To the control group (Vehicle), a PBS solution containing DMSO by 16% by mass was intranasally administered by the amount of 4 µL. The intranasal administration was performed 20 minutes before a test session of a forced swim test (FST) as set forth below.

### (Forced swim test)

A 7-week-old male ddY mouse is placed in a transparent plastic cylinder with a diameter of 18 cm and a height of 50 cm containing water at a temperature of 25°C±1°C to a height of 7 cm, and is allowed to swim for 15 minutes. Thereafter, the mouse is taken out from the cylinder and dried off with a towel, and returned to a cage. The procedure as mentioned above is referred to as a training session. After 24 hours of the completion of the training session, a 15-minute test session is performed in the same manner to the training session. The behavior of the mouse is recorded over the test session with a video camera. When the mouse is placed in the cylinder, it struggles to try to escape. When the mouse realizes that it cannot escape, the mouse gradually ceases to move giving up the escape. This situation is referred to as a state of immobility in which the mouse is in a depression-like state. The time during which the mouse is in a state of immobility (immobility time) is measured in the first 6 minutes from the start of the test session. The existence or non-existence of anti-depressant-like effects is determined by the length of the immobility time.

In the Examples described in the present specification, each of the forced swim test is performed by the aforementioned method.

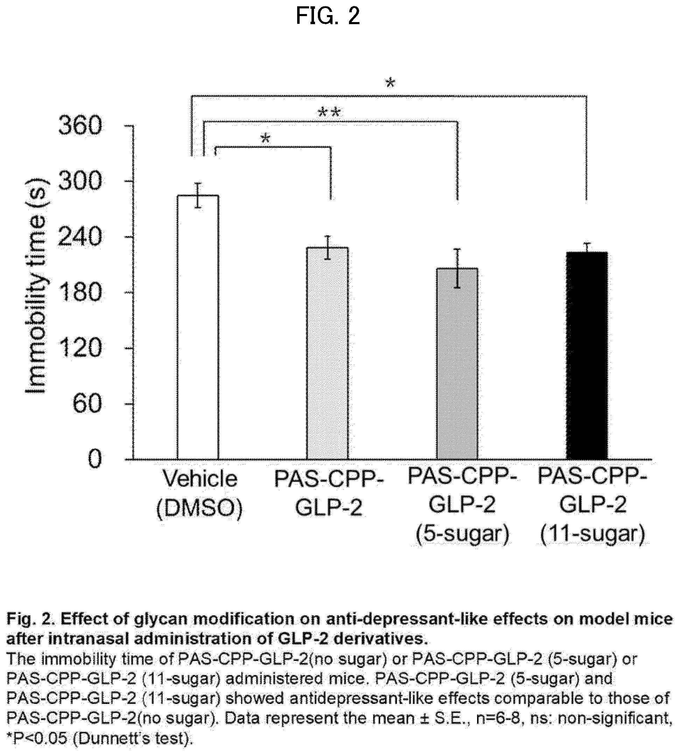

As shown in FIG. 2, all groups administered with GLP-2 derivatives exhibited a significantly shortened immobility time as compared with the control group, indicating the expression of anti-depressant effects, irrespective of the existence or non-existence of glycosylation.

The results suggest that a sugar chain (11-sugar) binding at the C-terminus of GLP-2 does not affect the pharmaceutical effects of the PAS-CPP-GLP-2 derivative.

### <Example 3: Evaluation on influence of PBS on pharmaceutical effects of glycosylated GLP-2 derivative>

A forced swim test (FST) was performed on mice in order to evaluate the influence of PBS on anti-depressant-like effects of a glycosylated GLP-2 derivative (C-terminus 11-sugar).

### (Preparation and administration of administration solution)

Administration solutions were prepared by mixing a PAS-CPP-GLP-2 derivative (no sugar) or a PAS-CPP-GLP-2 derivative (C-terminus 11-sugar) with PBS. In the solutions, the derivative with no sugar was suspended and the derivative with 11-sugar was completely dissolved. The concentration of each of the GLP-2 derivatives was adjusted to 0.6 nmol/4 µL.

After anesthetizing mice with isoflurane using an all-in-one small animal anesthetizer (MK-AT210D, Muromachi Kikai Co., Ltd., hereinafter the same), the solution was intranasally administered to the mice by the amount of 2 µL per nose cavity, 4 µL in total (0.6 nmol/mouse). To the control group (Vehicle), an equivalent amount of DMSO by 16% by mass was intranasally administered. The intranasal administration was performed 20 minutes before a test session of a forced swim test (FST) as set forth below.

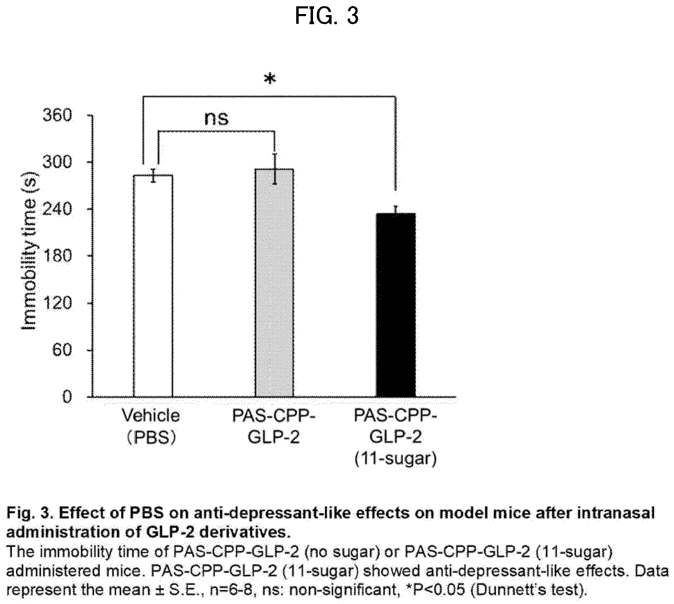

As shown in FIG. 3, the group administered with the PAS-CPP-GLP-2 derivative (no sugar) did not exhibit a significant anti-depressant-like effect as compared with the control group. The cause for the result is thought to be that the PAS-CPP-GLP-2 derivative did not dissolve in PBS. The group administered with the PAS-CPP-GLP-2 derivative (C-terminus 11- sugar) exhibited a significant anti-depressant-like effect as compared with the control group. The cause for the result is thought to be that the PAS-CPP-GLP-2 derivative dissolved in PBS by means of glycosylation.

The results suggest that the glycosylated PAS-CPP-GLP-2 derivative has an ability to express a centrally-acting activity even with an aqueous medium such as PBS. Accordingly, it is proved that there is no problem of initial concern, i.e., pharmaceutical effects may deteriorate due to an insufficient cell permeability of a derivative, due to an increase in water solubility caused by glycosylation.

### <Example 4: Evaluation on influence of glycosylation on migration of GLP-2 derivative to central nervous system>

The state of migration of the GLP-2 derivatives to the central nervous system was analyzed with an optical imaging apparatus in order to evaluate the influence of glycosylation on an ability of the GLP-2 derivative to migrate to the central nervous system.

### (Preparation of administration solution)

Administration solutions were prepared by completely dissolving the ICG-labeled PAS-CPP-GLP-2 derivative (no sugar) or the ICG-labeled PAS-CPP-GLP-2 derivative (C-terminus 11-sugar) in DMSO, and adjusting the final concentration of DMSO to 16% by adding PBS. While the PAS-CPP-GLP-2 derivative (11-sugar) was completely soluble in PBS, DMSO was used as a medium in order to conform the conditions to that of the PAS-CPP-GLP-2 derivative (no sugar). The concentration of the PAS-CPP-GLP-2 derivative (no sugar) or the PAS-CPP-GLP-2 derivative (11-sugar) was adjusted to 3.0 nmol/4 µL.

### (Evaluation on pathway to central nervous system with optical imaging apparatus)

After anesthetizing mice with isoflurane using an all-in-one small animal anesthetizer, the administration solutions or 16% DMSO as a control were intranasally administered to the mice by the amount of 2 µL per nose cavity, 4 µL in total.

In order to observe the migration to the brain over time, the brain was isolated 5 minutes, 10 minutes, 20 minutes, 60 minutes and 90 minutes after the intranasal administration, respectively, and subjected to infiltration fixation with 4% paraformaldehyde (4% PFA) solution overnight. Subsequently, a sagittal section with a thickness of 2 mm at the left and the right from the center of the brain was prepared using a brain matrix (RBM-2000S, ASI). The section was placed in a dish and analyzed with an optical imaging apparatus (Clairvivo OPT plus, Shimadzu Corporation) at an excitation wavelength of 785 nm, fluorescence wavelength of 849 nm, and an exposure time of 6 seconds.

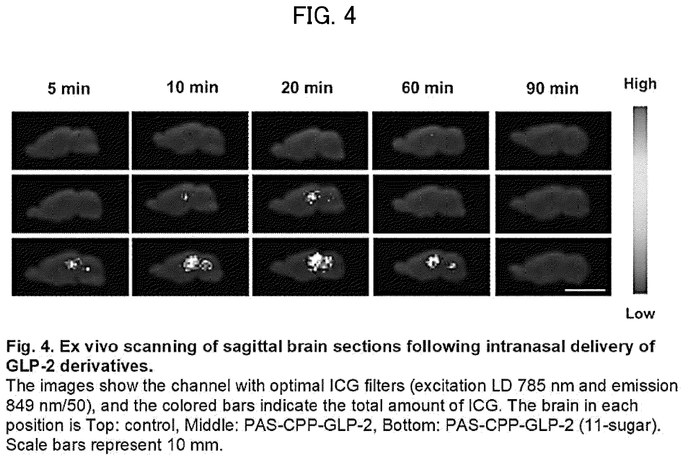

The results are shown in FIG. 4. Surprisingly, fluorescence was observed only in the case administered with the PAS-CPP-GLP-2 derivative (C-terminus 11-sugar) (Bottom) at a region around a posterior portion of the brain and the hippocampus/hypothalamic area at 5 minutes after the intranasal administration.

At 10 minutes after the intranasal administration, fluorescence was observed in each of the case administered with the PAS-CPP-GLP-2 derivative (no sugar) (Middle) and the case administered with the PAS-CPP-GLP-2 derivative (C-terminus 11-sugar). In the case administered with the PAS-CPP-GLP-2 derivative (no sugar), fluorescence was observed at a region around the hippocampus. In the case administered with the PAS-CPP-GLP-2 derivative (C-terminus 11-sugar), fluorescence was observed intensively at a region at which fluorescence was observed at 5 minutes after the intranasal administration, with an increased intensity.

At 20 minutes after the intranasal administration, fluorescence was observed in each of the case administered with the PAS-CPP-GLP-2 derivative (no sugar) and the case administered with the PAS-CPP-GLP-2 derivative (C-terminus 11-sugar). Fluorescence was observed in each case at a region around the hippocampus with an increased intensity as compared with 10 minutes after the administration, especially in the case of the PAS-CPP-GLP-2 derivative (C-terminus 11-sugar).

At 60 minutes after the intranasal administration, while fluorescence was not observed in the case administered with the PAS-CPP-GLP-2 derivative (no sugar), a relatively strong degree of fluorescence was observed in the case administered with the PAS-CPP-GLP-2 derivative (C-terminus 11- sugar).

At 90 minutes after the intranasal administration, fluorescence was not observed even in the case administered with the PAS-CPP-GLP-2 derivative (C-terminus 11- sugar), indicating the extinction thereof in the brain.

The results prove that the PAS-CPP-GLP-2 derivative (C-terminus 11-sugar) remains in the brain for a longer time period than the PAS-CPP-GLP-2 derivative (no sugar).

### <Example 5: Effect of glycosylation on amount of migration of GLP-2 derivative to brain>

The results of Example 4 prove that a greater amount of the PAS-CPP-GLP-2 derivative (C-terminus 11-sugar) migrates to the brain as compared with the PAS-CPP-GLP-2 (no sugar) from a qualitative viewpoint. In Example 5, the effect of glycosylation on the amount of the GLP-2 derivative to migrate to the brain is evaluated from a quantitative viewpoint by performing ELISA on the amounts of migration to the brain of the PAS-CPP-GLP-2 derivative (no sugar) and the PAS-CPP-GLP-2 derivative (11-sugar).

### (Quantification by ELISA on amount of migration to brain)

Mice were intranasally administered with 16% DMSO or the GLP-2 derivatives (6.0 nmol/mouse). The brain was isolated 20 minutes after the administration, and homogenized with BioMasher II (Nippi, Tokyo, Japan). A sample was prepared by performing centrifugal separation by 1000 xg (4°C) for 15 minutes and collecting a supernatant. The amount of the GLP-2 derivative in the sample was quantified using a GLP-2 ELISA kit. Specifically, a cleaning process including filling each well of a measurement plate with a cleaning fluid (350 µL) and suctioning the cleaning fluid with a pipette was performed three times. Subsequently, a labeled antigen solution (40 µL), the sample (25 µL) and a specific antibody solution (50 µL) were added in this order to the well and mixed. The measurement plate was tightly sealed and allowed to stand for 18 hours at 4°C. The cleaning process was performed three times, and a SA-HRP solution (100 µL) was added and shaken for 1 hour at room temperature (100 rpm). Immediately before the completion of the reaction, a color-former solution was prepared by dissolving an OPD tablet in a substrate solution (0.1 M citrate buffer solution containing 0.03% hydrogen peroxide). The cleaning process was performed five times, the color-former solution (100 µL) was added, and allowed to stand for 1 hour at room temperature under the light-shielding condition. Finally, an enzyme reaction stop solution (100 µL) was added and an absorbance at 490 nm was measured with ARVO (PerkinElmer Japan Co., Ltd., Kanagawa, Japan) to calculate the concentration of a GLP-2 derivative.

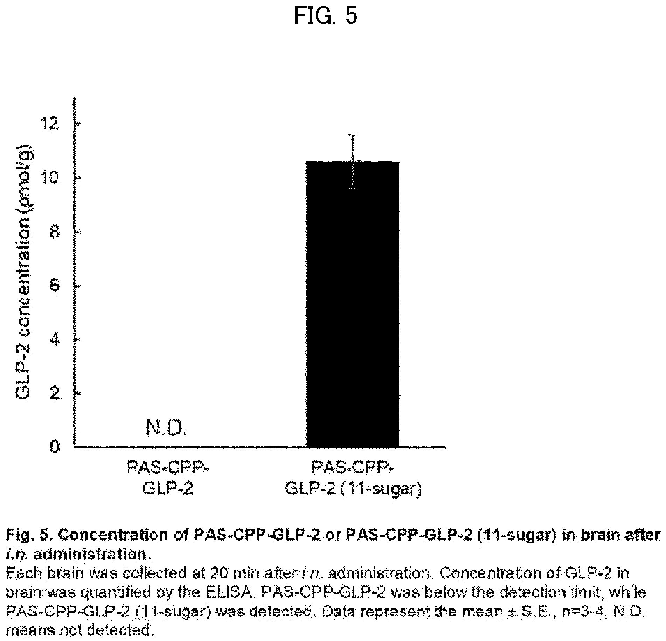

As a result, while the result of the group administered with the GLP-2 derivative without glycosylation was below the detection limit, the glycosylated GLP-2 derivative was detected by 10.598±0.998 pmol/g in the group administered with the glycosylated GLP-2 derivative (FIG. 5). The results indicate that the amount of migration of the GLP-2 derivative to the brain is increased by glycosylation.

### <Example 6: Evaluation on distribution in brain of glycosylated GLP-2 derivative>

The distribution in the brain of the glycosylated GLP-2 derivative (C-terminus 11-sugar) after the intranasal administration was evaluated by preparing frozen brain sections and subjecting the same to immunostaining. The brain sections were prepared from tissues around the regions considered as the action sites for GLP-2, i.e., the hippocampus (HIP) and the dorsomedial nucleus of the hypothalamus (DMH); and the regions including the olfactory nerve that is likely to serve as a migration pathway for GLP-2, i.e., the olfactory bulb (OB) and the pons/principal sensory nucleus of trigeminal nerve (Pr5).

### (Preparation of frozen brain section)

After anesthetizing 7-week-old male ddY mice with isoflurane using an all-in-one small animal anesthetizer, a 16% DMSO solution of the PAS-CPP-GLP-2 derivative (C-terminus 11-sugar) (3.0 nmol/4 µL) or 16% DMSO as a control was intranasally administered to the mice by the amount of 2 µL per nose cavity, 4 µL in total. The brain was isolated 5 minutes after the administration and 20 minutes after the administration, respectively.

The brain isolated 5 minutes after the intranasal administration was subjected to infiltration fixation with 4% PFA at 4°C overnight without performing perfusion fixation. The isolation of the brain 20 minutes after the intranasal administration was performed by the following process.

The mouse was fixed in a supine position under anesthesia with isoflurane, and the chest was incised. The tissue was fixed by perfusing with PBS, and subsequently with 4% PFA, from the left chamber of the heart throughout the whole body. The isolated brain was preserved in a 4% PFA solution. On the day following the isolation, the brain samples were substituted with 20% sucrose overnight (4°C), and further with 30% sucrose overnight (4°C). Thereafter, a frozen section with a thickness of 30 µm was prepared using a cryostat (CM3050S; Leica Microsystems).

### (Immunostaining)

The frozen brain section was mounted on a slide glass, and a circle was drawn around the section with a liquid blocker. A blocking buffer was added inside the circle and the section was subjected to blocking for 30 minutes at room temperature. Thereafter, the section was added with a first antibody solution including a GLP-2 polyclonal antibody, diluted by 200 times with a blocking solution, and subjected to incubation for 1 hour at room temperature. After rinsing three times with 1x PBS, the section was added with a second antibody solution including Alexa Fluor (trademark) 568 Goat Anti-Mouse IgG H&L, diluted by 500 times with a BSA/PBS solution, and subjected to incubation for 1 hour at room temperature. After rinsing three times with 1x PBS, the section was mounted with ProLong (trademark) Diamond Antifade Mountant. After confirming the solidification of the mountant, the section was subjected to fluorescent observation and imaging with a confocal laser microscope (TCS SP8; Leica) and a software (Leica Application Suite X Software; Leica).

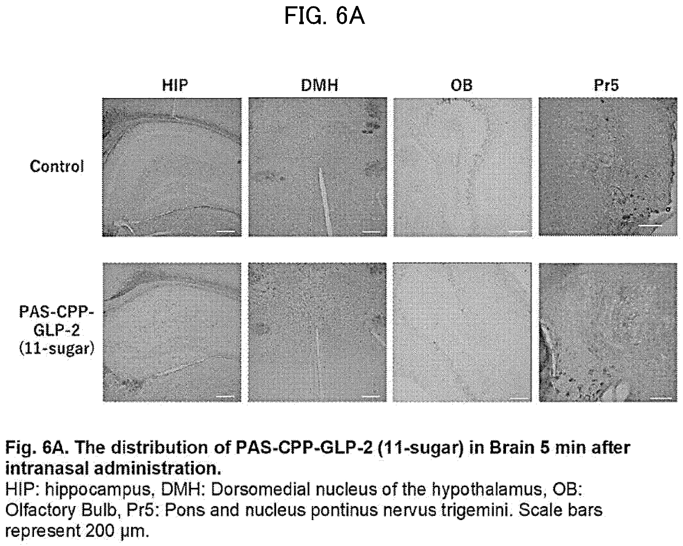

As shown in FIG. 6A, at 5 minutes after the intranasal administration, while fluorescence was observed intensely at a region around Pr5 in the case administered with the PAS-CPP-GLP-2 derivative (C-terminus 11-sugar), very little fluorescence was observed at the other regions. In the case administered with 16% DMSO as a control, very little fluorescence was observed at each of the regions.

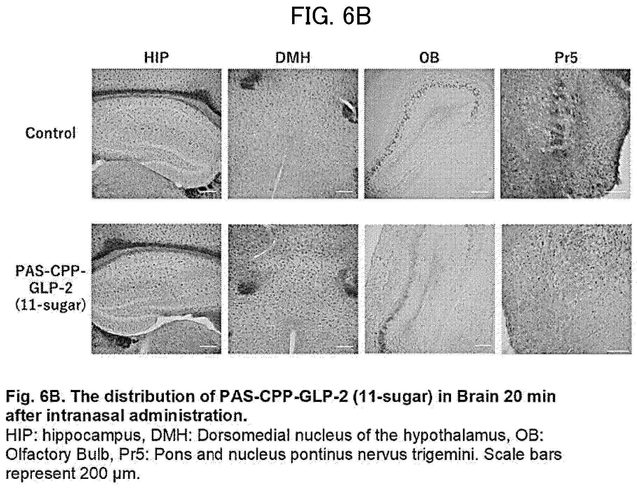

As shown in FIG. 6B, at 20 minutes after the intranasal administration, fluorescence was observed at HIP, and observed clearly at DMH in the case administered with the PAS-CPP-GLP-2 derivative (C-terminus 11-sugar). While florescence was observed also at Pr5 and OB, the intensity thereof was weak at OB. HIP and DMH are regions regarded as the action sites for GLP-2. In the case administered with 16% DMSO as a control, very little fluorescence was observed at each of the regions.

The results of the immunostaining conform to the length of time to express pharmaceutical effects, shown in the results of pharmaceutical tests in FIG. 2 and FIG. 3, supporting the idea that the glycosylated GLP-2 derivative reaches the action site and express pharmaceutical effects within 20 minutes after the intranasal administration. Further, the results suggest that the glycosylated GLP-2 derivative is delivered from the principal sensory nucleus of trigeminal nerve (Pr5) at the pons of brainstem to the hippocampus/hypothalamus as the action site, mainly through the trigeminal nerve in the respiratory epithelium, and expresses pharmaceutical effects.

The results overturn the theory regarded as the common knowledge in neuroscience, i.e., the rate of nervous axional transport is extremely low (50-400 mm/day in the fast case and 0.2-8 mm/day in the late case: Non-Patent Document 17).

### <Example 7: Effect of glycosylation on brain distribution of GLP-2 derivative >

While the results shown in FIG. 6A and FIG. 6B (Example 6) suggest that the glycosylated GLP-2 derivative is delivered from the principal sensory nucleus of trigeminal nerve (Pr5) at the pons of brainstem to the hippocampus and the hypothalamus as the action sites and expresses pharmaceutical effects, the effect of glycosylation on the distribution of the GLP-2 derivative in the brain still remains to be clarified. In Example 7, the effect of glycosylation on the distribution of the GLP-2 derivative in the brain is studied from the qualitative and quantitative viewpoints.

### (Preparation of frozen section)

Mice were intranasally administered with 16% DMSO or the GLP-2 derivatives (3.0 nmol/mouse), and the brain was isolated 5 minutes, 10 minutes, 20 minutes and 60 minutes after the administration.

The brain isolated 5 minutes or 10 minutes after the intranasal administration was subjected to infiltration fixation with 4% PFA at 4°C overnight without performing perfusion fixation. The isolation of the brain 20 minutes or 60 minutes after the intranasal administration was performed by the following process.

The mouse was fixed in a supine position under anesthesia with isoflurane, and the chest was incised. The tissue was fixed by perfusing with PBS, and subsequently with 4% PFA, from the left chamber of the heart throughout the whole body. The isolated brain was preserved in a 4% PFA solution. On the day following the isolation, the brain samples were substituted with 20% sucrose overnight (4°C), and further with 30% sucrose overnight (4°C). Thereafter, a frozen section with a thickness of 30 µm was prepared using a cryostat (CM3050S; Leica Microsystems).

Sections of the olfactory bulb (OB) including olfactory nerves, pons/principal sensory nucleus of trigeminal nerve (Pr5), hippocampus (HIP) and dorsomedial nucleus of the hypothalamus (DMH) were prepared by referring to the Nissil staining images and the brain map (Paxinos and Franklin, 2003).

### (Immunostaining and fluorescent observation in evaluation of brain distribution)

The frozen brain section was mounted on a slide glass, and a circle was drawn around the section with a liquid blocker. A blocking buffer was added inside the circle and the section was subjected to blocking for 30 minutes at room temperature. Thereafter, the section was added with a first antibody solution including a GLP-2 polyclonal antibody, diluted by 200 times with a blocking solution, and subjected to incubation for 1 hour at room temperature. After rinsing three times with 1x PBS, the section was added with a second antibody solution including Alexa Fluor (trademark) 568 Goat Anti-Mouse IgG H&L, diluted by 500 times with a BSA/PBS solution, and subjected to incubation for 1 hour at room temperature. After rinsing three times with 1x PBS, the section was mounted with ProLong (trademark) Diamond Antifade Mountant. After confirming the solidification of the mountant, the section was subjected to fluorescent observation and imaging with a confocal laser microscope (TCS SP8; Leica) and a software (Leica Application Suite X Software; Leica). Further, the fluorescent intensity in the image was calculated using the software and an average florescent intensity of each group was quantified.

The mice were intranasally administered with the PAS-CPP-GLP-2 (no sugar) or the PAS-CPP-GLP-2 (C-terminus 11-sugar). The brain was isolated 5 minutes, 20 minutes or 60 minutes after the administration, and a pharmaceutical distribution was observed at the olfactory bulb (OB) and the pons/principal sensory nucleus of trigeminal nerve (Pr5), which are regions including the olfactory nerve that is likely to serve as a migration route for GLP-2, and at the hippocampus (HIP) and the hypothalamus (DMH), which are regions regarded as the action sites for GLP-2.

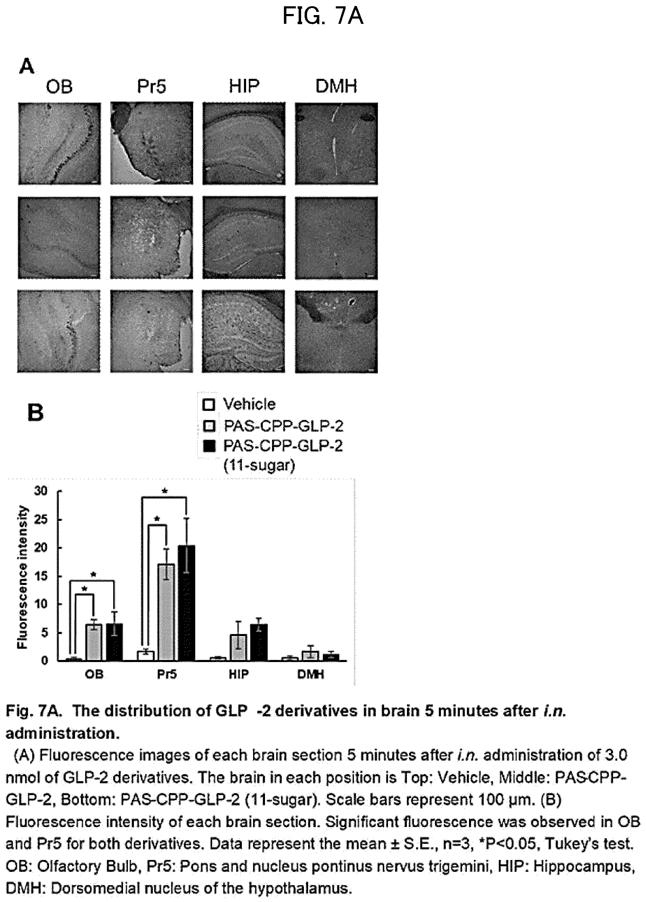

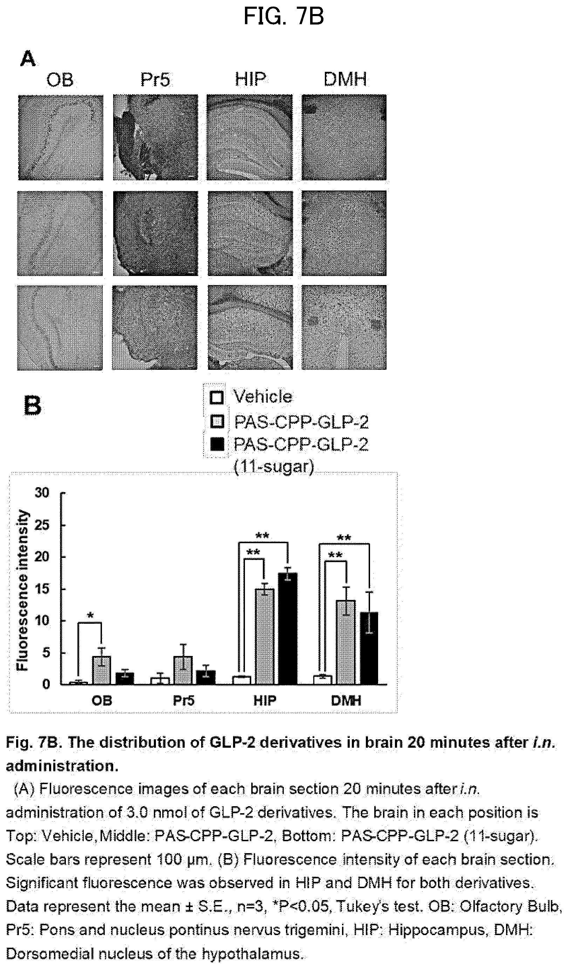

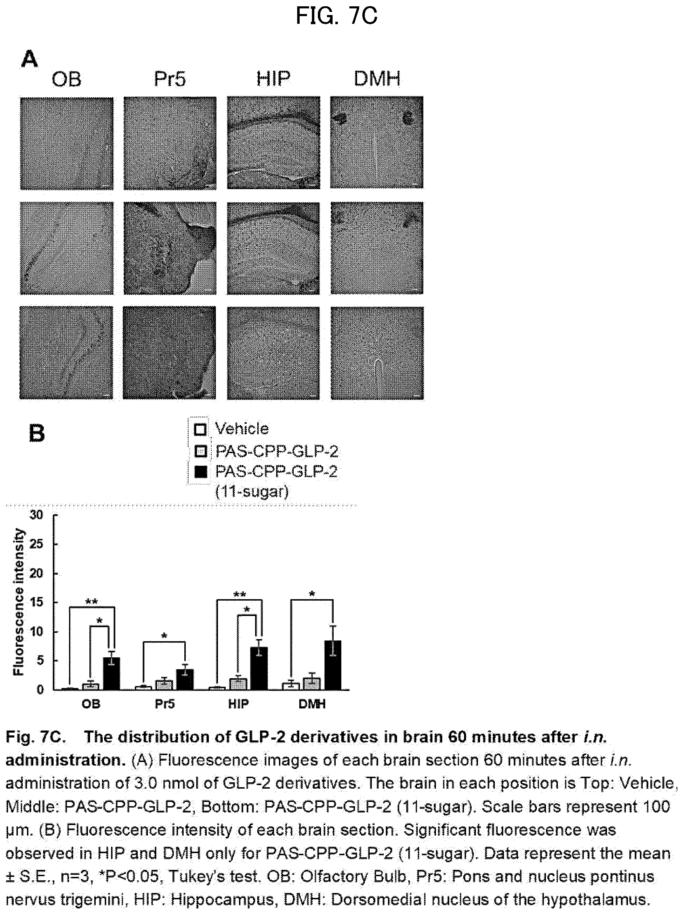

As a result, at 5 minutes after the administration, a significant degree of fluorescence was observed at OB and Pr5 including olfactory nerves, in each of the cases administered with the PAS-CPP-GLP-2 (no sugar) or the PAS-CPP-GLP-2 (C-terminus 11-sugar), with a greater intensity at Pr5 than OB (FIG. 7A).

At 20 minutes after the administration, while a significant degree of fluorescence was observed at OB in the case administered with the PAS-CPP-GLP-2 (no sugar), the intensity thereof at OB or Pr5 was weaker than the fluorescence observed 5 minutes after the administration in each of the cases administered with the PAS-CPP-GLP-2 (no sugar) or the PAS-CPP-GLP-2 (C-terminus 11-sugar). On the other hand, intense fluorescence was observed at HIP and DMH as the action sites for GLP-2 in each of the cases administered with the PAS-CPP-GLP-2 (no sugar) or the PAS-CPP-GLP-2 (C-terminus 11-sugar) (FIG. 7B).

The time period of 20 minutes after the administration conforms to the time for the GLP-2 derivative to express pharmaceutical effects, supporting the idea that each of the PAS-CPP-GLP-2 (no sugar) or the PAS-CPP-GLP-2 (C-terminus 11-sugar) is delivered to the action site and expresses pharmaceutical effects.

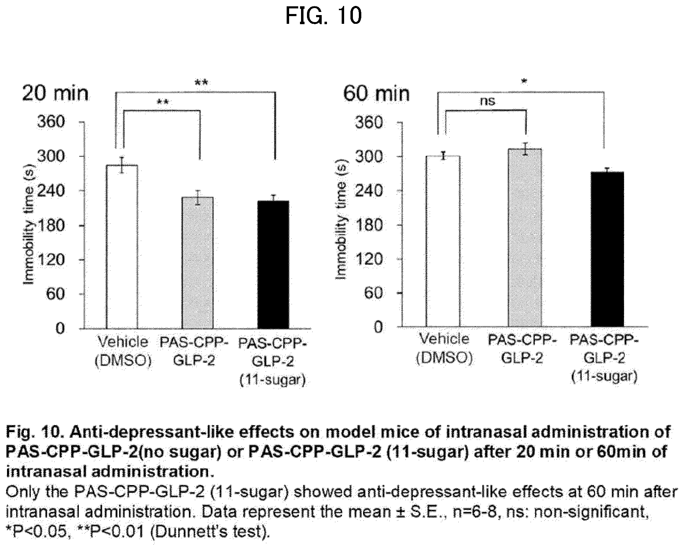

At 60 minutes after the administration, while a significant degree of fluorescence was observed at OB and Pr5 in the case administered with the PAS-CPP-GLP-2 (C-terminus 11-sugar), the intensity thereof was weaker than the fluorescence observed at 5 minutes after the administration. At HIP and DMH as the action sites for GLP-2, while fluorescence was not observed in the case administered with the PAS-CPP-GLP-2 (no sugar), a significant degree of fluorescence was observed in the case administered with the PAS-CPP-GLP-2 (C-terminus 11-sugar) (FIG. 7C). The results suggest that the glycosylation has an effect of enhancing the continuity of pharmaceutical effects of the GLP-2 derivative, as proved in Example 10 (FIG. 10).

### <Example 8: Observation of section of trigeminal nerve>

After anesthetizing 7-week-old male ddY mice with isoflurane using an all-in-one small animal anesthetizer, a 16% DMSO solution of the PAS-CPP-GLP-2 derivative (C-terminus 11-sugar) labeled with FITC (3.0 nmol/4 µL) or 16% DMSO as a control was intranasally administered to the mice by the amount of 2 µL per nose cavity, 4 µL in total. The trigeminal nerve was isolated 5 minutes after the administration.

The trigeminal nerve was subjected to infiltration fixation with 4% PFA overnight. On the day following the isolation, the trigeminal nerve was substituted with 20% sucrose overnight (4°C), and further with 30% sucrose overnight (4°C). Thereafter, a frozen section with a thickness of 20 µm was prepared using a cryostat (CM3050S; Leica Microsystems).

The frozen section was mounted on a slide glass, and a circle was drawn around the section with a liquid blocker. To the inner side of the circle, 10 mM CuSO₄/CH₃COONH₄ having an ability to suppress auto-fluorescence was added to immerse the section for 15 minutes. After rinsing three times with 1x PBS, a blocking buffer was added inside the circle and the section was subjected to blocking for 30 minutes at room temperature. Thereafter, the section was added with a first antibody solution including Neuro-Chrom (trade name) Pan Neuronal Marker Antibody-Rabbit, diluted by 1000 times with a blocking solution, and subjected to incubation for 2 hours at room temperature. After rinsing three times with 1x PBS, the section was added with a second antibody solution including Alexa Fluor (trademark) 568 Goat Anti-Mouse IgG H&L, diluted by 500 times with a BSA/PBS solution, and subjected to incubation for 1 hour at room temperature. After rinsing three times with 1x PBS, the section was mounted with ProLong (trademark) Diamond Antifade Mountant. After confirming the solidification of the mountant, the section was subjected to fluorescent observation and imaging with a confocal laser microscope (TCS SP8; Leica) and a software (Leica Application Suite X Software; Leica).

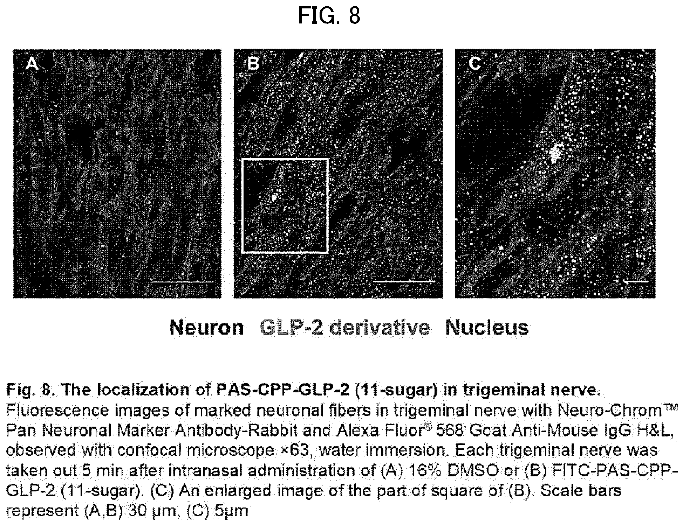

In FIG. 8, the image indicated as A represents the section of trigeminal nerve isolated after the administration of 16% DMSO; the image indicated as B represents the section of trigeminal nerve isolated after the administration of the 16% DMSO solution of the PAS-CPP-GLP-2 derivative (C-terminus 11-sugar); and the image indicated as C represents an enlarged image of a square portion in B.

As shown in FIG. 8, the section of trigeminal nerve isolated after the administration of the PAS-CPP-GLP-2 derivative (C-terminus 11-sugar) expresses yellow fluorescence (the brightest portions in the image), indicating overlapping of green fluorescence representing the PAS-CPP-GLP-2 derivative and red fluorescence representing neural fibers, in addition to a large amount of green fluorescence representing the PAS-CPP-GLP-2 derivative.

The results suggest that the PAS-CPP-GLP-2 derivative (C-terminus 11-sugar) is delivered to the central nervous system through the trigeminal nerve in which trigeminal neural fibers are included.

### <Example 9: Evaluation on migration of glycosylated GLP-2 derivative to trigeminal lemniscus after intranasal administration>

As proved in Example 8, the PAS-CPP-GLP-2 derivative (C-terminus 11-sugar) is delivered from the trigeminal nerve at the respiratory epithelium to the principal sensory nucleus of trigeminal nerve (Pr5), which is the projection of the trigeminal nerve.

In Example 9, whether the PAS-CPP-GLP-2 derivative (C-terminus 11-sugar) migrates to the trigeminal lemniscus, which is a pathway that connects Pr5 with the ventral posterior medial nucleus (VPM) of the thalamus, was studied.

### (Observation of section of trigeminal lemniscus)

Mice were intranasally administered with 16% DMSO or the glycosylated GLP-2 derivative (3.0 nmol/mouse). The brain was isolated 15 minutes after the administration, and was subjected to infiltration fixation with 4% PFA overnight. On the day following the isolation, the brain was substituted with 30% sucrose overnight (4°C). Thereafter, a frozen section of trigeminal lemniscus with a thickness of 30 µm was prepared using a cryostat (CM3050S; Leica Microsystems, WetZlar, Germany).

The frozen section was mounted on a slide glass, and was subjected to blocking for 30 minutes at room temperature with a blocking buffer. Thereafter, the section was added with a first antibody solution including Neuro-Chrom (trade name) Pan Neuronal Marker Antibody-Rabbit, diluted with a blocking buffer (1:500), and subjected to incubation overnight (4°C). After rinsing three times with 1x PBS, the section was added with a first antibody solution including a GLP-2 polyclonal antibody diluted with a BSA/PBS solution (1:200), and subjected to incubation for 2 hours at room temperature. After rinsing three times with 1x PBS, the section was added with a second antibody solution including Alexa Fluor (trademark) 568 Goat Anti-Mouse IgG H&L, diluted by 1000 times with a 1% B SA/PB S solution and DAPI (40 ng/ml), and subj ected to incubation for 1 hour at room temperature. After rinsing three times with 1x PBS, the section was mounted and the trigeminal lemniscus was subjected to fluorescent observation with a confocal laser microscope (TCS SP8; Leica, WetZlar, Germany) and a software (Leica Application Suite X Software; Leica, WetZlar, Germany).

The trigeminal lemniscus, which is a pathway that connects the principal sensory nucleus of trigeminal nerve Pr5 with the ventral posterior medial nucleus (VPM) of the thalamus, was observed.

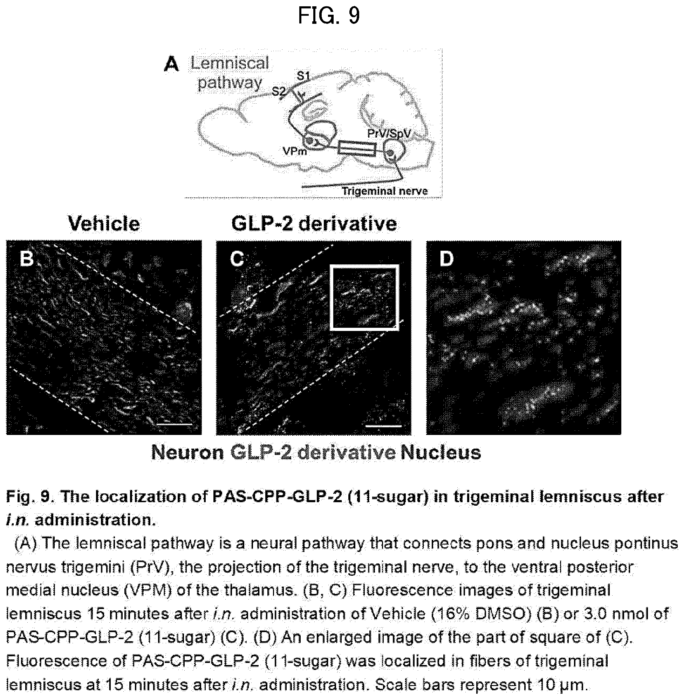

In the case of the PAS-CPP-GLP-2 derivative (11-sugar), a large amount of fluorescence representing the drug was observed in the nerve bundle that constitutes the trigeminal lemniscus (FIG. 9, C) and yellow fluorescence, in which green fluorescence representing neural fibers and red fluorescence representing the drug overlap each other, was observed (FIG. 9, D).

The results shown in FIG. 8 and FIG. 9 strongly suggest that the intranasally-administered glycosylated PAS-CPP-GLP-2 derivative migrates from the respiratory epithelium to the principal sensory nucleus of trigeminal nerve (Pr5) via the trigeminal nerve, and further to the thalamus as the action site via the trigeminal lemniscus.

### <Example 10: Evaluation on pharmaceutical continuity of glycosylated GLP-2 derivative>

As proven in Example 5, localization of the GLP-2 derivative at the hippocampus and the thalamus as action sites continues. Since the PAS-CPP-GLP-2 derivative (C-terminus 11-sugar) may maintain the anti-depressant-like effects as its centrally-acting activity, continuity of the anti-depressant-like effects was evaluated.

After anesthetizing 7-week-old male ddY mice with isoflurane using an all-in-one small animal anesthetizer, 16% DMSO was administered to the control group (Vehicle), and a 16% DMSO solution (0.6 nmol/4 µL) of the PAS-CPP-GLP-2 derivative (C-terminus 11-sugar) or the PAS-CPP-GLP-2 derivative (no sugar) was administered to the GLP-2 administration groups, by the amount of 2 µL per nose cavity, 4 µL in total. After 20 minutes of the intranasal administration, the mice were subjected to a test session of a forced swim test.

The mice assigned to a different control group and different GLP-2 administration groups were subjected to a test session of a forced swim test 60 minutes after the intranasal administration.

As shown in FIG. 10, when the forced swim test was performed 20 minutes after the intranasal administration (left graph), the immobility time in each of the group administered with the PAS-CPP-GLP-2 derivative (C-terminus 11-sugar) or the PAS-CPP-GLP-2 derivative (no sugar) was significantly shortened as compared with the control group (Vehicle), indicating the expression of anti-depressant-like effects.

When the forced swim test was performed 60 minutes after the intranasal administration (right graph), a significant difference is shown only between the group administered with the PAS-CPP-GLP-2 derivative (11-sugar) and the control group (Vehicle), indicating the expression of anti-depressant-like effects.

The results of Example 10, combined with the results of Example 5, suggest that the glycosylated GLP-2 derivative remains at the hippocampus and the thalamus as the action sites for a longer time than the PAS-CPP-GLP-2 derivative that is not glycosylated, and continues to express anti-depressant-like effects.

### <Example 11: Evaluation on enhancing pharmaceutical effects of glycosylated GLP-2 derivative>

Since a greater amount of the PAS-CPP-GLP-2 derivative (C-terminus 11-sugar) tends to localize at the hippocampus/thalamus as the action sites than the PAS-CPP-GLP-2 derivative (no sugar), as proven in Example 6, it is likely that the PAS-CPP-GLP-2 derivative (C-terminus 11-sugar) expresses anti-depressant-like effects with a smaller amount of administration than the PAS-CPP-GLP-2 derivative (no sugar). Therefore, whether the PAS-CPP-GLP-2 derivative (C-terminus 11-sugar) expresses anti-depressant-like effects with a smaller amount of administration than the PAS-CPP-GLP-2 derivative (no sugar) was studied.

After anesthetizing 7-week-old male ddY mice with isoflurane using an all-in-one small animal anesthetizer, 16% DMSO was administered to the control group (Vehicle), and a 16% DMSO solution (0.6 nmol/4 µL) of the PAS-CPP-GLP-2 derivative (C-terminus 11-sugar) or the PAS-CPP-GLP-2 derivative (no sugar) was administered to the GLP-2 administration groups, by the amount of 2 µL per nose cavity, 4 µL in total. After 20 minutes of the intranasal administration, the mice were subjected to a test session of a forced swim test.

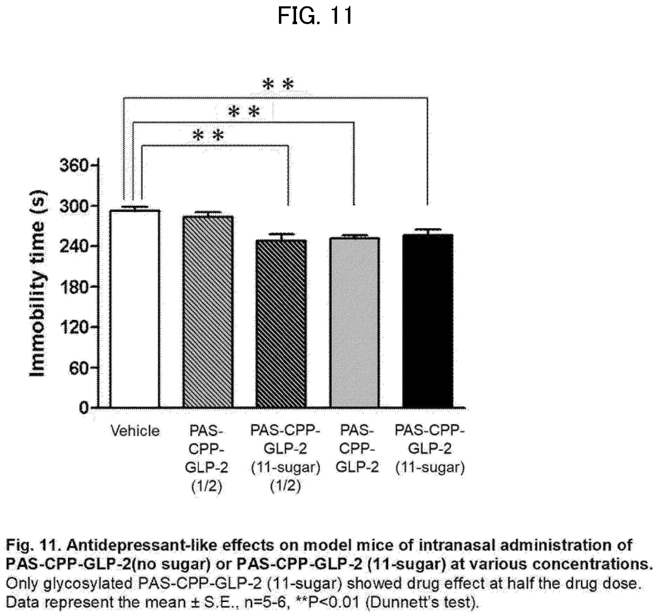

As shown in FIG. 11, while the group administered with the PAS-CPP-GLP-2 derivative (no sugar) expressed anti-depressant-like effects at an amount of 0.6 nmol/mouse, the group did not express a significant degree of anti-depressant-like effects at an amount of 0.3 nmol/mouse. On the other hand, the group administered with the PAS-CPP-GLP-2 derivative (C-terminus 11-sugar) expressed anti-depressant-like effects at an amount of 0.3 nmol/mouse, and a degree thereof was equivalent to that expressed at an amount of 0.6 nmol/mouse.

The results indicate that the PAS-CPP-GLP-2 derivative (C-terminus 11-sugar) expresses anti-depressant-like effects at a smaller administration amount than a PAS-CPP-GLP-2 derivative (no sugar), suggesting that the glycosylation has an effect of enhancing the pharmaceutical effects of a neuropeptide derivative.

### <Example 12: Evaluation on cellular uptake mechanism of glycosylated GLP-1 derivative to neurons>

Considering a mechanism of cellular uptake of the glycosylated GLP-2 derivatives is an important issue in a process of searching neuropeptide derivative applicable for clinical use. Therefore, an experiment was conducted to investigate a mechanism of the glycosylated GLP-2 derivatives to be taken into neuron cells referred to as Neuro 2A.

Specifically, whether or not the glycosylated GLP-2 derivative (C-terminus 11-sugar) was taken into Neuro 2A by inducing macropinocytosis was studied using EIPA (5-(N-ethyl-N-isopropyl)-amiloride), a specific inhibitor of micropinocytosis.

### (Preparation of treatment solution for cells)

In order to sufficiently inhibit the cellular uptake pathway of micropinocytosis, Neuro 2A cells were added with a culture solution containing EIPA for 30 minutes prior to exposing the cells to the GLP-2 derivative (pre-treatment). The culture solution was prepared by dissolving EIPA in DMSO and diluting the same with 10% DMEM to adjust a final concentration to 1%.

After the pre-treatment, a solution prepared by adding EIPA dissolved in DMSO to the FITC-PAS-CPP-GLP-2 derivative (C-terminus 11-sugar), and adjusting the final concentrations to 0.045 µg/µL (derivative) and 100 µM (EIPA) with the culture solution, was added to the Neuro 2A cells.

As for the exposure to the cells in the control group, a DMSO solution of the PAS-CPP-GLP-2 derivative (C-terminus 11-sugar) (0.045 µg/µL as the derivative) was prepared.

### (Study on cellular uptake pathway)

Neuro 2A cells were incubated for 24 hours in a 12-well plate seeded at an amount of 2 x 10⁵ cells/well, and it was confirmed that the cells were completely adhering to the 12-well plate.

Subsequently, to the cells of the group added with EIPA, a solution containing EIPA (pre-treatment) was added at an amount of 500 µL/well and allowed to stand in an incubator for 20 minutes. Thereafter, an EIPA solution containing the FITC-PAS-CPP-GLP-2 derivative (C-terminus 11-sugar) was added at an amount of 500 µL/well, and allowed to stand. To the cell for the control group, the same treatment was performed using a solution not containing EIPA.

After 30 minutes from the start of exposure, the cell was collected to a tube after rinsing with 1x PBS by 500 µL/well and treating with trypsin. The tube was subjected to centrifugal treatment at 1000 rpm for 5 minutes, added with FACS buffer at an amount of 1000 µL/well to form a cell suspension, and further subjected to centrifugal treatment at 1000 rpm for 5 minutes. The tube was further added with FACS buffer at an amount of 1000 µL/well to suspend the cells therein. The suspension was filtered with a nylon mesh filter and allowed to stand in an ice bath until the measurement.

In the measurement, the fluorescence intensity of FITC was measured using an automatic cell analyze system BD FACSCalibur (trademark) (Becton, Dickinson and Company) and the analysis was performed using FlowJo (FlowJo Software).

Macropinocytosis is a system that causes cellular uptake by means of reconstruction of an actin skeleton and formation of a ruffling structure of fluid plasma membranes. Since endosomal vesicles with a size of greater than 1 µm are produced, the system of macropinocytosis is expected to achieve efficient cellular uptake (Non-Patent Document 16).

In view of the foregoing, whether or not the cellular uptake was caused by macropinocytosis was investigated by measuring the amount of cellular uptake using the cells treated with EIPA as a specific inhibitor for macropinocytosis.

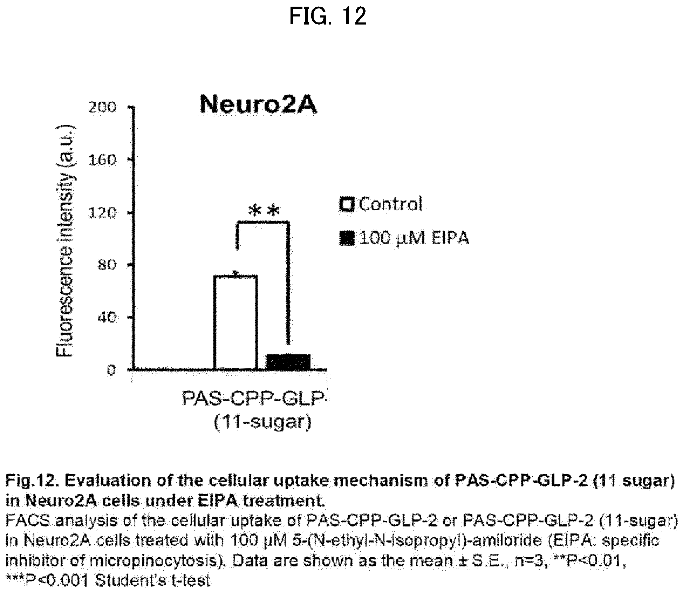

As a result, as shown in FIG. 12, the amount of the PAS-CPP-GLP-2 derivative (C-terminus 11-sugar) taken into Neuro 2A cells was significantly decreased under the existence of EIPA. The results prove that a glycosylated neuropeptide derivative is taken into cells by way of macropinocytosis.

### <Example 13: Evaluation on effects of reducing medicinally effective amount of glycosylated GLP-2 derivative>

The GLP-2 derivative is a peptide that is poorly soluble in water, and not only the improvement in solubility but also the enhancement of pharmaceutical effects is achieved by glycosylation. However, it is not necessarily true that any neuropeptide derivative added with PAS-CPP is poorly soluble in water. For example, since PAS-CPP-GLP-1 derivatives are highly soluble in water, experiments for evaluating the pharmaceutical effects are conducted by dissolving the PAS-CPP-GLP-1 derivative in PBS. Therefore, in order to support the usefulness of glycosylation, it is important to prove that the pharmaceutical effects are enhanced by glycosylation not only in the case of PAS-CPP-GLP-2 derivatives, which are poorly soluble with respect to water, but also in the case of PAS-CPP-GLP-1 derivatives, which are highly soluble with respect to water. In view of the foregoing, experiments were conducted to investigate whether or not a glycosylated GLP-1 derivative expresses a greater pharmaceutical effect than a GLP-1 derivative that is not glycosylated.

### (Preparation of GLP-1 derivative)

A PAS-CPP-GLP-1 derivative (C-terminus 11-sugar) was prepared in the same manner as the PAS-CPP-GLP-2 derivative (C-terminus 11-sugar), except that GLP-1 was used instead of GLP-2 as the neuropeptide sequence. The configuration of the GLP-1 derivative is described below.

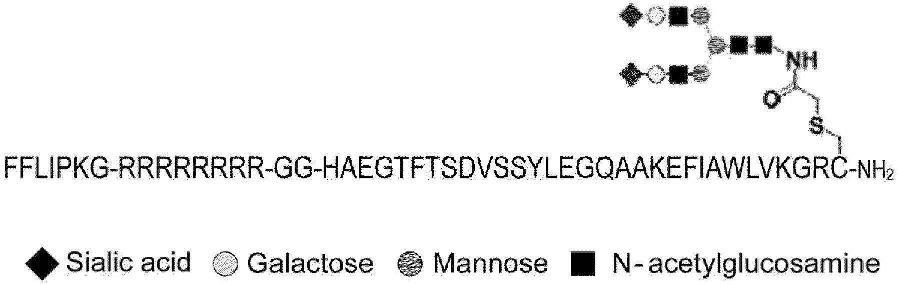

A PAS-CPP-GLP-1 derivative (no sugar) was prepared in the same manner as the PAS-CPP-GLP-2 (no sugar), except that GLP-1 was used instead of GLP-2 as the neuropeptide sequence. The configuration of the GLP-1 derivative is described below.

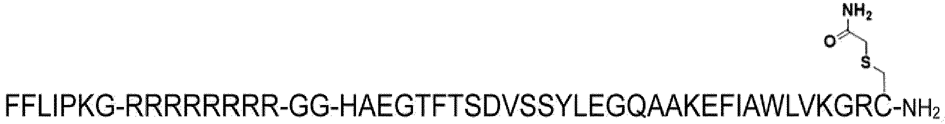

### (Preparation of LPS-induced amnesia model mice)

An LPS solution was prepared by dissolving lipopolysaccharide (SIGMA-Aldrich) in 0.01M PBS to adjust a concentration to 10 µg/5 µL.

After anesthetizing 7-week-old male ddY mice with isoflurane, the mice in the LPS administration group were administered with the LPS solution by an intraventricular manner by the amount of 10 µg/mouse. Specifically, the LPS solution (5 µL) was administered using a 50-µL syringe (Hamilton (R) GASTIGHT (R) syringe, 1700) and an intracerebral needle (28G x 3mm, Natsume Seisakusho) over 15 seconds in consideration of a pressure difference. To the mice in the control group, 0.01M PBS (5 µL) was administered.

### (Preparation and administration of administration solution)

Since the PAS-CPP-GLP-1 derivative (no sugar) is soluble in PBS, administration solutions were prepared by dissolving the PAS-CPP-GLP-1 derivative (no sugar) or the PAS-CPP-GLP-1 derivative (C-terminus 11-sugar) in PBS (0.2 nmol/4 µL).

After anesthetizing with isoflurane, the mice were administered with the administration solution by the amount of 2 µL per nose cavity, 4 µL in total (0.2 nmol/mouse). The mice in the control group and the LPS group were administered with PBS by the amount of 2 µL per nose cavity, 4 µL in total. The administration was performed 20 minutes prior to performing a Y-maze test as described below.

### (Y-maze test)

A Y-shaped maze made of black acrylic plates (angles between arms: 120°) was used as an apparatus for the test. The size of each arm is 10 cm at an upper section, 3 cm at a bottom, 12 cm at a height, and 40 cm at a length. A mouse is placed at an edge of the Y-shaped maze, and the arms in which the mouse moves within 8 minutes are recorded in order.

The percent alternation was calculated by dividing the number of times of entries three different arms in a consecutive manner by a value obtained by deducting 2 from the total number of times of entries (total arm entries) and multiplying the quotient by 100. The alternation is used as an index for the leaning/memory behavior.

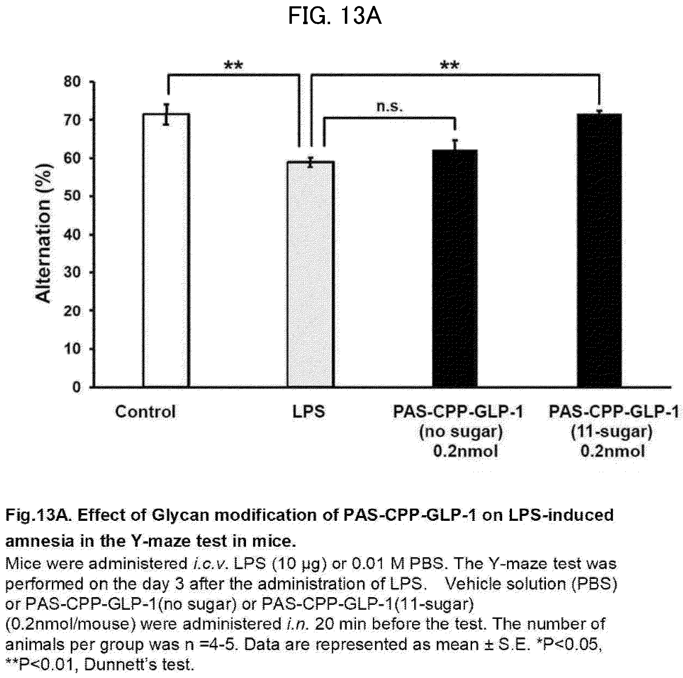

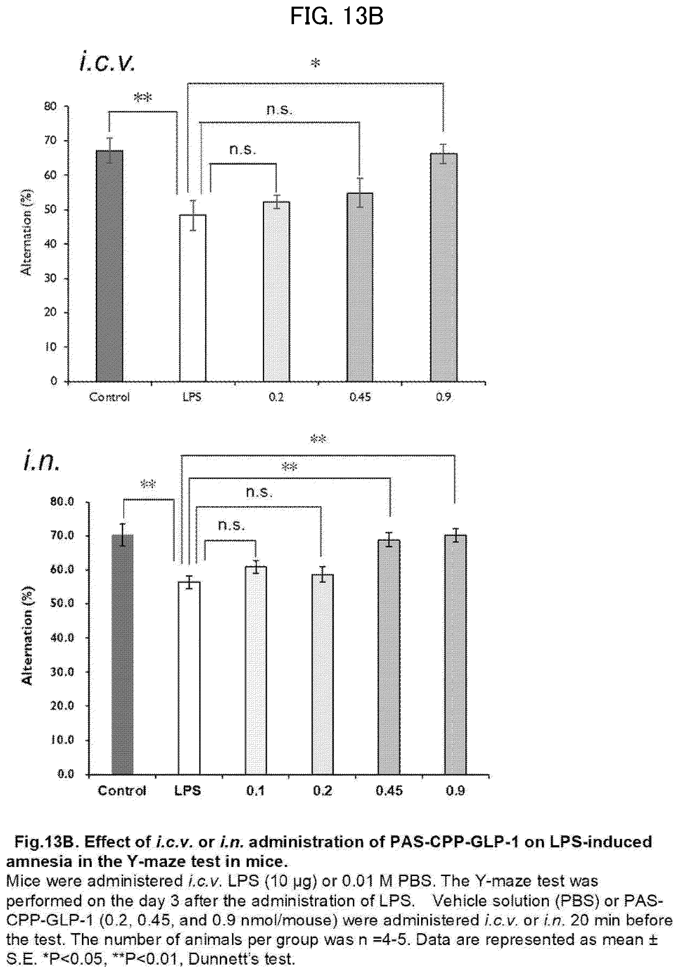

As shown in FIG. 13A, while the group administered with the PAS-CPP-GLP-1 derivative (no sugar) did not show a significant effect of improving the learning/memory functions as compared with the LPS group in which only PBS was administered, the group administered with the PAS-CPP-GLP-1 derivative (C-terminus 11-sugar) showed a significant effect of improving the learning/memory functions as compared with the LPS group in which only PBS was administered. It is noted that the group administered with the PAS-CPP-GLP-1 derivative (no sugar) tends to show a significant effect of improving the learning/memory functions as the amount of the PAS-CPP-GLP-1 derivative (no sugar) for administration is increased, as shown in FIG. 13B,

A Y-maze test was conducted in which the administration amount of the PAS-CPP-GLP-1 derivative (no sugar) was changed to the amounts as shown in FIG. 12 (nmol/mouse), and the administration was conducted in an intranasal manner and in an intraventricular manner, respectively.

As shown in FIG. 13B, while the group administered with the PAS-CPP-GLP-1 derivative (no sugar) showed an alternation of over 60% at 0.9 nmol/mouse in the case of intraventricular administration (i.c.v.), the group showed an alternation of over 60% at 0.45 nmol/mouse in the case of intranasal administration (i.n.)

The results suggest that the PAS-CPP-GLP-1 derivative (no sugar) is delivered to the central nervous system by the intranasal administration more efficiently than the intraventricular administration.

Further, the results shown in FIG. 13A and FIG. 13B indicate that the PAS-CPP-GLP-1 derivative (11-sugar) expresses an equal or more degree of alternation at an administration amount of less than a quarter (0.2 nmol/mouse) with respect to the PAS-CPP-GLP-1 derivative (no-sugar), even in a case that the PAS-CPP-GLP-1 derivative (11-sugar) is administered in an intranasal manner and the PAS-CPP-GLP-1 derivative (no-sugar) is administered in an intraventricular manner. These results are not what a skilled person could have conceived of based on the common knowledge, and should be appreciated as a support for the usefulness of the glycosylated neuropeptide derivative of the present invention.

Since the PAS-CPP-GLP-1 derivative (no sugar) is highly soluble with respect to water, unlike the PAS-CPP-GLP-2 derivative (no sugar), the derivative is dissolved in PBS rather than DMSO in the experiments for the evaluation of pharmaceutical effects. In other words, the experiments reveal that the glycosylation imparts, to a peptide derivative, enhanced pharmaceutical effects (i.e., reduced pharmaceutically effective amount) even if the peptide derivative is highly water-soluble. These results indicate the effectiveness of glycosylation for peptide derivatives having a functional sequence (PAS-CPP), irrespective of a degree of water solubility thereof, widening a range of application of the glycosylation to peptides having a functional sequence (PAS-CPP).

### <Example 14: Study on neuropeptide derivative glycosylated at N-terminus>

### (Preparation of glycosylated GLP-1 derivative (N-terminus 11-sugar))

A PAS-CPP-GLP-1 derivative (no sugar), in which an endosomal-escape accelerating sequence (PAS: FFLIPKG), a cell-penetration accelerating sequence (CPP: RRRRRRRR), a spacer sequence including a cysteine residue (GCG) and an amino acid sequence derived from GLP-1 as a neuropeptide sequence are disposed in this order from the N-terminus side, was prepared. The configuration of the GLP-1 derivative is described below.

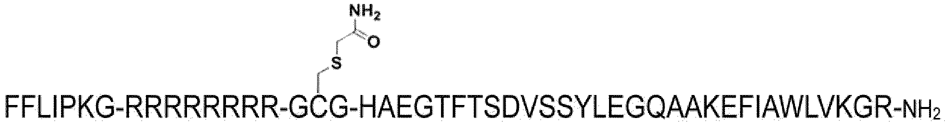

Subsequently, a PAS-CPP-GLP-1 derivative glycosylated at the N terminus side (N-terminus 11-sugar) was obtained by binding a sugar chain (11-sugar) to the cysteine residue in the spacer sequence. The configuration of the glycosylated GLP-1 derivative is described below.

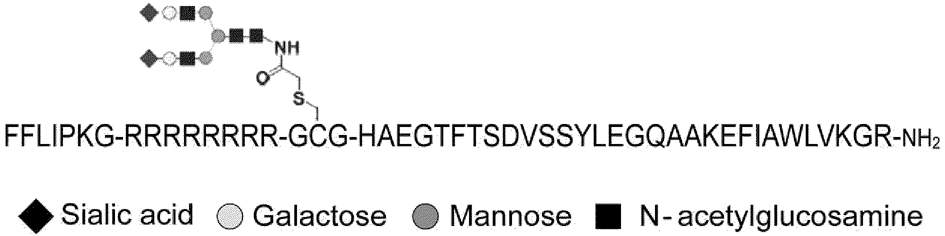

### (Preparation of administration solution)

Administration solutions were prepared by completely dissolving the ICG-labeled PAS-CPP-GLP-1 derivative (no sugar) or the ICG-labeled PAS-CPP-GLP-1 derivative (N-terminus 11-sugar) in DMSO, and adjusting the final concentration of DMSO to 16% by adding PBS.

While the PAS-CPP-GLP-1 derivative (no sugar) is completely soluble in PBS, the ICG-labeled PAS-CPP-GLP-1 derivative does not dissolve in PBS. Meanwhile, the ICG-labeled PAS-CPP-GLP-1 derivative that is glycosylated at the N terminus (N-terminus 11-sugar) is completely soluble in PBS. Therefore, DMSO was used as a medium in order to conform the conditions. The concentration of the PAS-CPP-GLP-1 derivative (no sugar) or the PAS-CPP-GLP-2 derivative (N-terminus 11-sugar) was adjusted to 3.0 nmol/4 µL, respectively.

### (Evaluation on pathway to central nervous system with optical imaging apparatus)

After anesthetizing 7-week-old male ddY mice with isoflurane using an all-in-one small animal anesthetizer, the administration solutions or 16% DMSO as a control was intranasally administered to the mice by the amount of 2 µL per nose cavity, 4 µL in total.

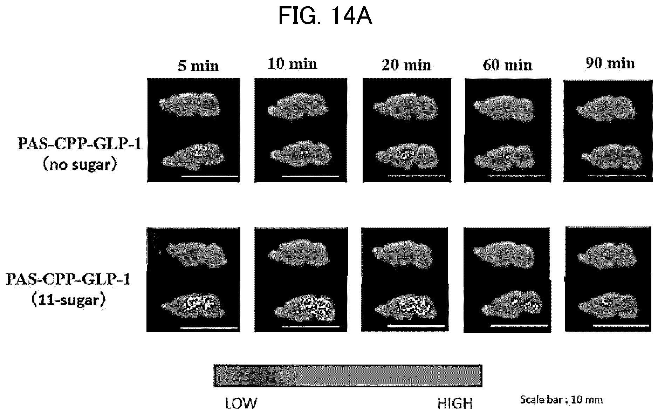

In order to observe the migration to the brain over time, the brain was isolated 20 minutes after the intranasal administration, and subjected to infiltration fixation with 4% paraformaldehyde (4% PFA) solution overnight. Subsequently, a sagittal section with a thickness of 2 mm at the left and the right from the center of the brain was prepared using a brain matrix (RBM-2000S, ASI). The section was placed in a dish and analyzed with an optical imaging apparatus (Clairvivo OPT plus, Shimadzu Corporation) at an excitation wavelength of 785 nm, fluorescence wavelength of 849 nm, and an exposure time of 6 seconds. The results are shown in FIG. 14A.

As shown in FIG. 14A, the sample administered with the PAS-CPP-GLP-1 derivative (N-terminus 11-sugar) exhibits a greater amount of migration to the central nervous system than the sample administered with the PAS-CPP-GLP-1 derivative (no sugar), suggesting that the ability of the PAS-CPP-GLP-1 derivative to migrate to the central nervous system is improved by glycosylation.

### (Quantification by ELISA on amount of migration to brain)

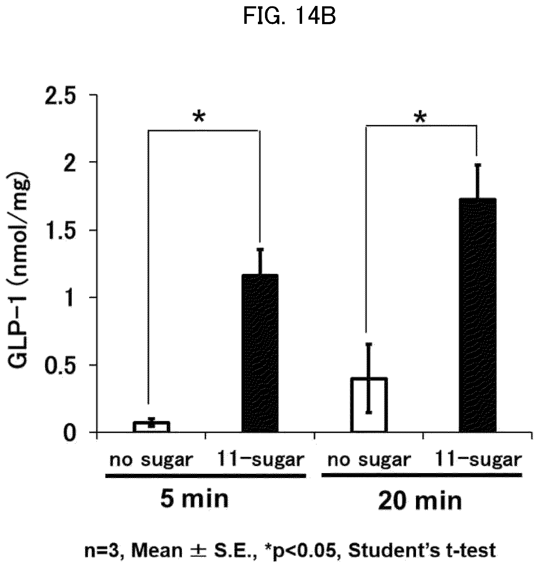

Mice were intranasally administered with 16% DMSO or the GLP-1 derivatives (3.6 nmol/mouse). The brain was isolated 5 minutes or 20 minutes after the administration, and homogenized with BioMasher II (Nippi, Tokyo, Japan). A sample was prepared by performing centrifugal separation by 1000 xg for 15 minutes and collecting a supernatant. The amount of the GLP-1 derivative in the sample was quantified using a GLP-1 ELISA kit. Specifically, a cleaning process including filling each well of a measurement plate with a cleaning fluid (350 µL) and suctioning the cleaning fluid with a pipette was performed three times. Subsequently, a labeled antigen solution (40 µL), the sample (25 µL) and a specific antibody solution (50 µL) were added in this order to the well and mixed. The measurement plate was tightly sealed and allowed to stand for 18 hours at 4°C. The cleaning process was performed three times, and a SA-HRP solution (100 µL) was added and allowed to permeate for 1.5 hours at room temperature (60 rpm). Immediately before the completion of the reaction, a color-former solution was prepared by dissolving an OPD tablet in 25 mL of a substrate solution (0.1 M citrate buffer solution containing 0.03% hydrogen peroxide). The cleaning process was performed five times, the color-former solution (100 µL) was added, and allowed to stand for 1 hour at room temperature under the light-shielding condition. Finally, an enzyme reaction stop solution (100 µL) was added and an absorbance at 490 nm was measured with ARVO (PerkinElmer Japan Co., Ltd., Kanagawa, Japan) to calculate the concentration of the GLP-1 derivative. The results are shown in FIG. 14B.

As shown in FIG. 14B, the sample administered with the PAS-CPP-GLP-1 derivative (N-terminus 11-sugar) exhibits a greater concentration than the sample administered with the PAS-CPP-GLP-1 derivative (no sugar), suggesting that the ability of the PAS-CPP-GLP-1 derivative to migrate to the central nervous system is improved by glycosylation.

### (Evaluation on effects of reducing medicinally effective amount of GLP-1 derivative glycosylated at N terminus)

### (Preparation of LPS-induced amnesia model mice)

An LPS solution was prepared by dissolving lipopolysaccharide (SIGMA-Aldrich) in 0.01M PBS to adjust a concentration to 10 µg/5 µL.

After anesthetizing 7-week-old male ddY mice with isoflurane, the mice in the LPS administration group were administered with the LPS solution by intraventricular administration by the amount of 10 µg/mouse. Specifically, the LPS solution (5 µL) was administered using a 50-µL syringe (Hamilton (R) GASTIGHT (R) syringe, 1700) and an intracerebral needle (28G x 3mm, Natsume Seisakusho) over 15 seconds in consideration of a pressure difference. To the mice in the control group, 0.01M PBS (5 µL) was administered.

### (Preparation and administration of administration solution)

Since the PAS-CPP-GLP-1 derivative (no sugar) is soluble in PBS, administration solutions were prepared by dissolving the PAS-CPP-GLP-1 derivative (no sugar) or the PAS-CPP-GLP-1 derivative (N-terminus 11-sugar) in PBS, respectively.

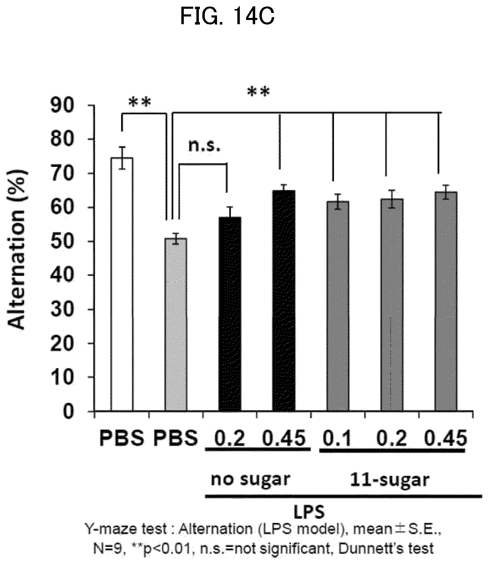

After anesthetizing with isoflurane, the mice were administered with the administration solution by the amount of 2 µL per nose cavity, 4 µL in total (0.2 nmol/mouse). The mice in the control group and the LPS group were administered with PBS by the amount of 2 µL per nose cavity, 4 µL in total. The administration was performed 20 minutes prior to performing a Y-maze test. The Y-maze test was performed in the same manner as Example 13. The results are shown in FIG. 14C.

As shown in FIG. 4C, while the sample administered with the PAS-CPP-GLP-1 derivative (no sugar) exhibited a pharmaceutical effect at 0.45 nmol/mouse, the sample administered with the PAS-CPP-GLP-1 derivative (N-terminus 11-sugar) exhibited a pharmaceutical effect at 0.1 nmol/mouse, suggesting that the glycosylation decreases the medicinally effective amount of the PAS-CPP-GLP-1 derivative (N-terminus 11-sugar).

### (Evaluation on continuity in pharmaceutical effect of GLP-1 derivative glycosylated at N-terminus)

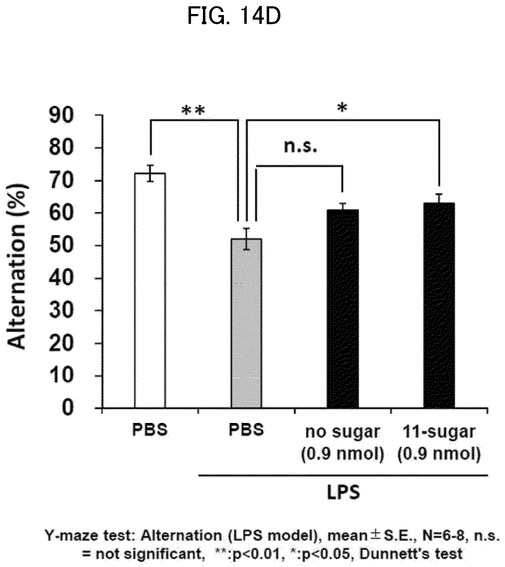

The Y-maze test was performed in the same manner as supplemental Example 3, except that the test was performed 60 minutes after the administration. The results are shown in FIG. 14D.

As shown in FIG. 14D, while the sample administered with the PAS-CPP-GLP-1 derivative (no sugar) did not exhibit a pharmaceutical effect at 60 minutes after the administration, the sample administered with the PAS-CPP-GLP-1 derivative (N-terminus 11- sugar) exhibited a pharmaceutical effect at 60 minutes after the administration, suggesting that the glycosylation improves the continuity of pharmaceutical effects of the PAS-CPP-GLP-1 derivative (N-terminus 11- sugar).

### (Evaluation on rapidity of action of GLP-1 derivative glycosylated at N-terminus)

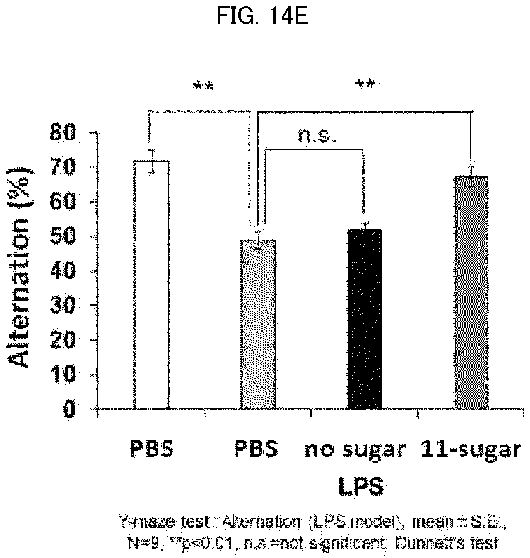

The Y-maze test was performed in the same manner as supplemental Example 3, except that the test was performed 5 minutes after the administration. The results are shown in FIG. 14E.

As shown in FIG. 14E, while the sample administered with the PAS-CPP-GLP-1 derivative (no sugar) did not exhibit a pharmaceutical effect at 5 minutes after the administration, the sample administered with the PAS-CPP-GLP-1 derivative (N-terminus 11-sugar) exhibited a pharmaceutical effect at 5 minutes after the administration, suggesting that the glycosylation improves the rapidity of action of the PAS-CPP-GLP-1 derivative (N-terminus 11-sugar).

### (Evaluation on effect of reducing medicinally effective amount of GLP-2 derivative glycosylated at N-terminus)

A PAS-CPP-GLP-2 derivative (no sugar) and a PAS-CPP-GLP-2 derivative (N-terminus 11-sugar) having the following configurations were prepared by changing GLP-1 to GLP-2.

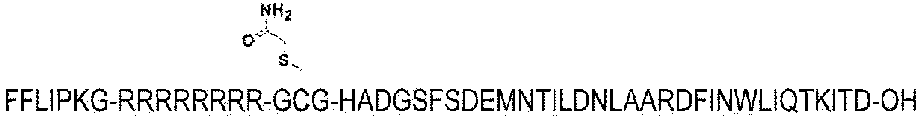

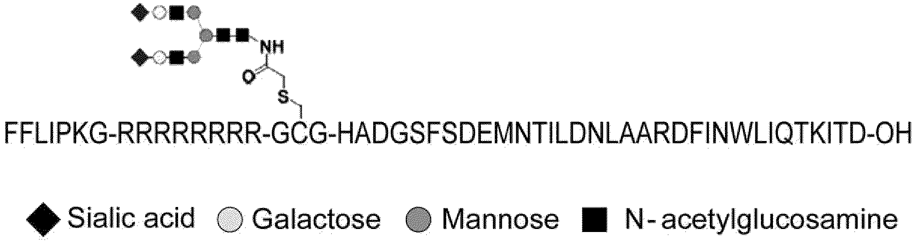

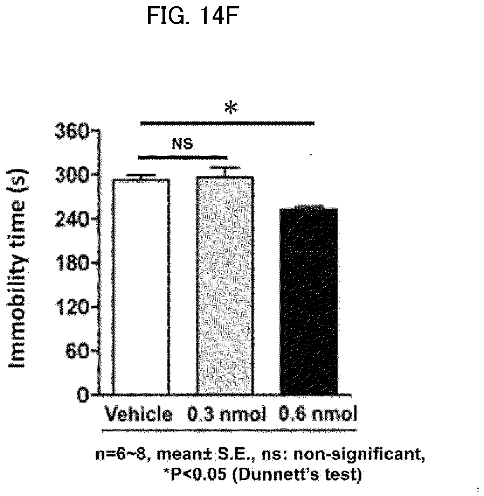

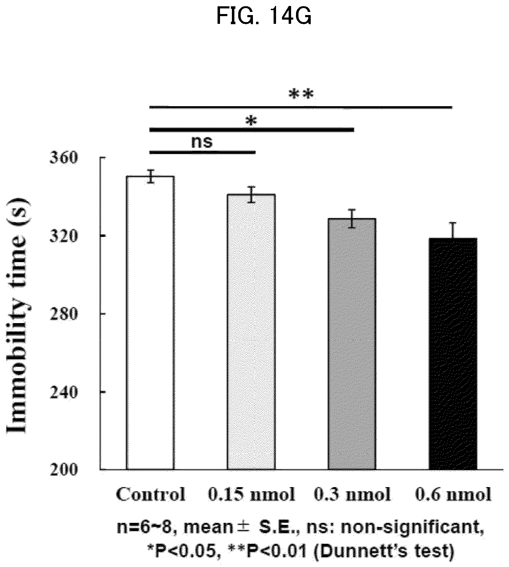

Administration solutions were prepared by completely dissolving the PAS-CPP-GLP-2 derivative (no sugar or N-terminus 11-sugar) in DMSO and adding 16% DMSO thereto. After anesthetizing the mice with isoflurane using an all-in-one small animal anesthetizer, the solution was intranasally administered to the mice. Specifically, the intranasal administration was performed by contacting a tip of the anesthetizer to a nasal cavity in a horizontal manner such that the solution was inhaled by spontaneous respiration, by the amount of 2 µL per nose cavity, 4 µL in total. To the control group (Vehicle), 16% DMSO was intranasally administered by the amount of 4 µL. The intranasal administration was performed 20 minutes before a test session of a forced swim test (FST). The results of the forced swim test are shown in FIG. 14F and FIG. 14G.

As shown in FIG. 14F and FIG. 14G, while the sample administered with the PAS-CPP-GLP-2 derivative (no sugar) exhibited a pharmaceutical effect at an amount of 0.6 mmol, the sample administered with the PAS-CPP-GLP-2 derivative (N-terminus 11-sugar) exhibited a pharmaceutical effect at an amount of 0.3 nmol, suggesting that the glycosylation decreases the medicinally effective amount of the PAS-CPP-GLP-2 derivative (N-terminus 11-sugar).

### <Example 15: Study on GLP-2 derivative glycosylated via linker (N-terminus 11-sugar, C6 linker: C6)>

### (Preparation of glycosylated GLP-2 derivative)

A PAS-CPP-GLP-2 derivative (N-terminus 11-sugar), in which an endosomal-escape accelerating sequence (PAS: FFLIPKG), a cell-penetration accelerating sequence (CPP: RRRRRRRR), a spacer sequence including a cysteine residue (GCG) and an amino acid sequence derived from GLP-1 as a neuropeptide sequence are disposed in this order from the N-terminus side, and a sugar chain (11-sugar) was bound to the cysteine residue of the spacer sequence, was prepared. The configuration of the GLP-2 derivative is shown below.

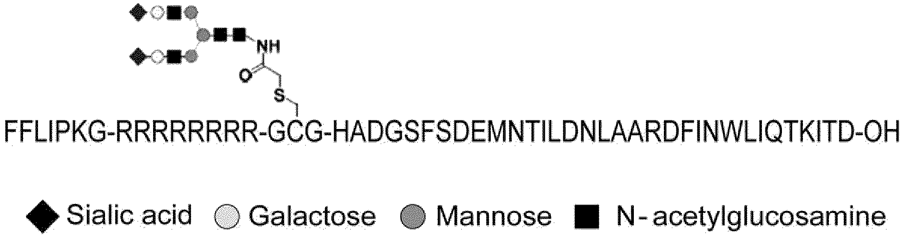

A PAS-CPP-GLP-2 derivative (N-terminus 11-sugar, C6 linker: C6) in which the cysteine residue in the spacer sequence was changed to a lysine residue (K), and a glycosylation molecule including a sugar chain (11-sugar) and an alkylene group with a carbon number of 6 as a linker, was bound to the lysine residue, was prepared. The configuration of the GLP-2 derivative is shown below.

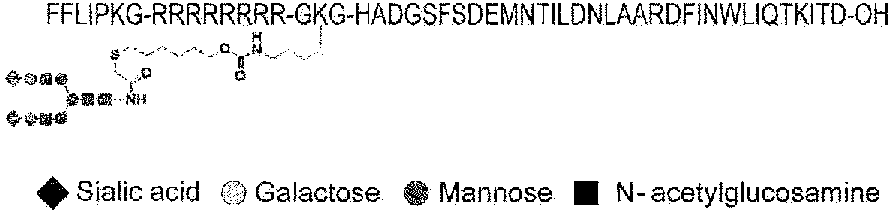

### (Evaluation on cellular uptake amount into neuron cells>

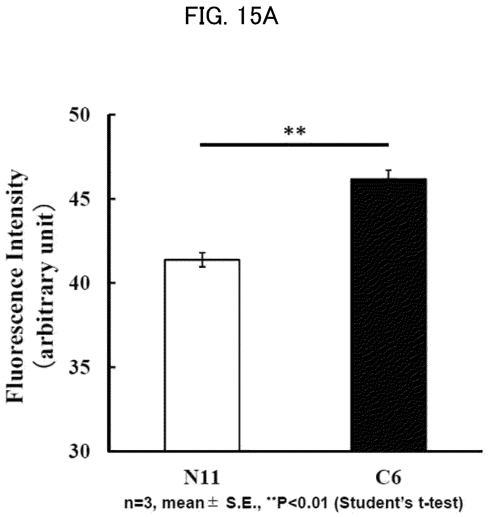

Neuro 2A cells were seeded in a 12-well plate at an amount of 2 x 10⁵ cells/well, and incubated for 24 hours. After confirming that the cells were completely adhering to the 12-well plate, a solution containing the derivative of each kind was added at an amount of 500 µL/well and allowed to stand in an incubator. The well plate was taken out from the incubator at 30 minutes after starting the exposure, rinsed with 1x PBS by an amount of 500 µL/well, and the cells were collected in a tube by performing trypsin treatment. After the tube was subjected to centrifugal treatment five times at 1000 rpm, a cell suspension was prepared by adding FACS buffer by an amount of 1000 µL/tube, and the tube was further subjected to centrifugal treatment five times at 1000 rpm. The cells were further suspended by adding FACS buffer by an amount of 1000 µL/tube, filtered with a nylon mesh filter, and allowed to stand in an ice bath until the measurement. The measurement was performed by an automatic cell analysis system BD FACSCalibur^{™} (Becton, Dickinson and Company, New Jersey, USA) with FlowJo (Software, USA) for the analysis. The results are shown in FIG. 15A. In FIG. 15A, "N11" indicates without a linker and "C6" indicates with a C6 linker.

As shown in FIG. 15A, the glycosylated GLP-2 derivative (N-terminus 11-sugar, C6 linker: C6), in which a sugar chain is bound to a peptide derivative via a linker, exhibited an increase in the amount of uptake into cells, as compared with the case of the glycosylated GLP-2 derivative (N-terminus 11-sugar, without linker: N11), in which a sugar chain is bound to a peptide derivative without a linker.

### (Evaluation on effect of reducing medicinally effective amount)

Administration solutions were prepared by completely dissolving the derivatives in PBS. After anesthetizing mice with isoflurane using an all-in-one small animal anesthetizer, the solution was intranasally administered to the mice. Specifically, the intranasal administration was performed by contacting a tip of the anesthetizer to a nasal cavity in a horizontal manner such that the solution was inhaled by spontaneous respiration, by the amount of 2 µL per nose cavity, 4 µL in total. To the control group (Vehicle), PBS was intranasally administered by the amount of 4 µL.

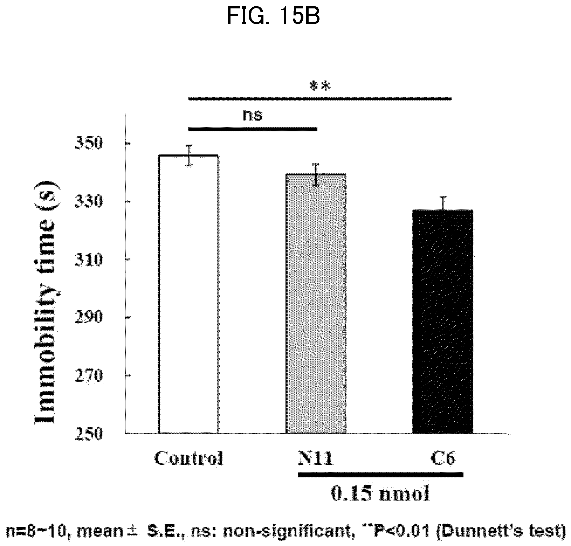

The intranasal administration was performed 20 minutes before starting a test session of a forced swim test (FST). The results of the forced swim test are shown in FIG. 15B.

As shown in FIG. 15B, the glycosylated GLP-2 derivative (N-terminus 11-sugar, C6 linker: C6), in which a sugar chain is bound to a peptide derivative via a linker, showed a decrease in immobility time, indicating the expression of anti-depressant-like effects, as compared with the glycosylated GLP-2 derivative (N-terminus 11-sugar, without linker: N11), in which a sugar chain is bound to a peptide derivative without a linker.

### (Evaluation on neural activity at action site for anti-depressant-like effects (DMH: dorsomedial nucleus of the hypothalamus)>

Brain samples were isolated 20 minutes after the intranasal administration of PBS or the glycosylated GLP-2 derivatives to mice (0.15 nmol/mouse), and subjected to infiltration fixation with 4% PFA at 4°C overnight. On the day following the isolation, the brain samples were substituted with 30% sucrose overnight (4°C). Thereafter, frozen sections of the hippocampus and the dorsomedial nucleus of the hypothalamus with a thickness of 40 µm were prepared using a cryostat (CM3050S; Leica Microsystems, WetZlar, Germany).

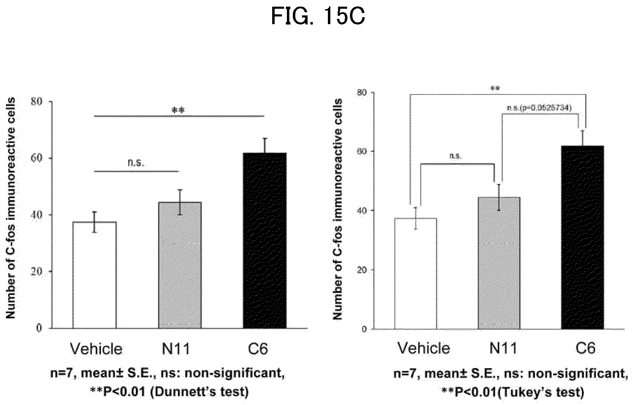

The samples were placed on a slide glass, added with BLOXALL^{™} Blocking Solution, and subjected to blocking for 10 minutes at room temperature. After rinsing three times with 1x PBS, the samples were added with Normal Horse Serum with 0.3% TritonX-100 and subjected to permeabilization for 20 minutes at room temperature. The samples were added with a primary antibody solution including Anti-c-Fos antibody diluted with 2.5% BSA/PBS solution (1:10000) and incubated overnight at 4°C. After rinsing with 1x PBS three times, the samples were added with a secondary antibody solution including biotinylated horse anti-mouse/rabbit IgG secondary antibody, and incubated for 2 hours at room temperature. After rinsing with 1x PBS three times, the samples were added with VECTSTAIN Elite ABC Reagent and incubated for 1.5 hours at room temperature. After rinsing with 1x PBS three times, the samples were added with ImmPACT^{™} DAB EqV solution and incubated for 10 minutes at room temperature. After rinsing with purified water, the samples were mounted and the expression of c-Fos in the HIP and the DMH was observed using an optical microscope (BIO-REVO BZ-9000; Keyence, Osaka, Japan). The c-Fos positive cells in the obtained images were counted and quantified by calculating the average number of respective groups. The results are shown in FIG. 15C.

As shown in FIG. 15C, the glycosylated GLP-2 derivative (N-terminus 11-sugar, C6 linker: C6), in which a sugar chain is bound to a peptide derivative via a linker, exhibited a significant increase in the expression of c-Fos at the action site for anti-depressant-like effects, reflecting the anti-depressant-like effects observed in the forced swim test.

### (Effects of binding position of sugar chain on amount of migration to central nerve system)

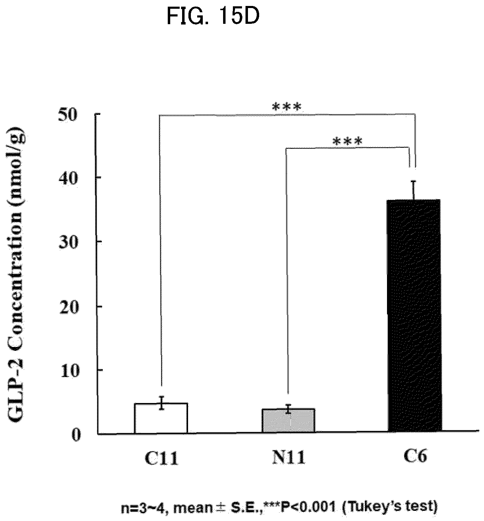

Mice were intranasally administered with the PAS-CPP-GLP-2 derivative prepared in Example 1 (C-terminus 11-sugar, no linker, C11), the PAS-CPP-GLP-2 derivative (N-terminus 11-sugar, no linker, N11), and the PAS-CPP-GLP-2 derivative glycosylated via a C6 alkylene group (N-terminus 11-sugar, with C6 linker, C6), respectively (6.0 nmol/mouse). The brain was isolated 20 minutes after the administration, and homogenized with BioMasher II (Nippi, Tokyo, Japan). A sample was prepared by performing centrifugal separation by 1000 xg (4°C) for 15 minutes and collecting a supernatant. The amount of the GLP-2 derivative in the sample was quantified using a GLP-2 ELISA kit. Specifically, a cleaning process including filling each well of a measurement plate with a cleaning fluid (350 µL) and suctioning the cleaning fluid with a pipette was performed three times. Subsequently, a labeled antigen solution (40 µL), the sample (25 µL) and a specific antibody solution (50 µL) were added in this order to the well and mixed. The measurement plate was tightly sealed and allowed to stand for 18 hours at 4°C. The cleaning process was performed three times, and a SA-HRP solution (100 µL) was added and shaken for 1 hour at room temperature (100 rpm). Immediately before the completion of the reaction, a color-former solution was prepared by dissolving an OPD tablet in 25 mL of a substrate solution (0.1 M citrate buffer solution containing 0.03% hydrogen peroxide). The cleaning process was performed five times, the color-former solution (100 µL) was added, and allowed to stand for 1 hour at room temperature under the light-shielding condition. Finally, an enzyme reaction stop solution (100 µL) was added and an absorbance at 490 nm was measured with ARVO (PerkinElmer Japan Co., Ltd., Kanagawa, Japan) to calculate the concentration of a GLP-2 derivative. The results are shown in FIG. 15D. In FIG. 15D, C11 indicates the PAS-CPP-GLP-2 derivative (C-terminus 11-sugar, no linker), N11 indicates the PAS-CPP-GLP-2 derivative (N-terminus 11-sugar, no linker), and C6 indicates the PAS-CPP-GLP-2 derivative (N-terminus 11-sugar, with C6 linker), respectively.

As shown in FIG. 15D, C6, in which a sugar chain is bound to a peptide derivative via a linker, exhibited an increase in the amount of migration to the central nerve system as compared with C11 or N11, in which a sugar chain is bound to a peptide derivative without a linker.

### <Reference Example: Evaluation on effects of addition of cell-penetration accelerating sequence and endosomal-escape accelerating sequence>

A GLP-2 derivative referred to as CPP-GLP-2, in which a cell-penetration accelerating sequence (CPP: RRRRRRRR), a spacer sequence (GG) and an amino acid sequence derived from GLP-2 as a neuropeptide sequence are disposed in this order; a GLP-2 derivative referred to as PAS-GLP-2, in which an endosomal-escape accelerating sequence (PAS: FFLIPKG), a spacer sequence (GG) and an amino acid sequence derived from GLP-2 as a neuropeptide sequence are disposed in this order; and a GLP-2 derivative referred to as PAS-CPP-GLP-2, in which an endosomal-escape accelerating sequence (PAS: FFLIPKG), a cell-penetration accelerating sequence (CPP: RRRRRRRR), a spacer sequence (GG) and an amino acid sequence derived from GLP-2 as a neuropeptide sequence are disposed in this order were prepared by a known method.

The GLP-2 derivatives were not modified with a sugar chain in order to eliminate an influence of glycosylation.

Administration solutions were prepared by completely dissolving the GLP-2 derivatives or GLP-2 in DMSO and adding PBS to adjust the final concentration of DMSO to 16% by mass. The concentration of the GLP-2 derivatives or GLP-2 was adjusted to 0.6 nmol/4 µL, respectively.

After anesthetizing 7-week-old male ddY mice with isoflurane using an all-in-one small animal anesthetizer, the administration solutions were intranasally administered by the amount of 2 µL per nose cavity, 4 µL in total (0.6 nmol/mouse). The mice in the control group (Vehicle) were administered with 16% DMSO by the amount of 4 µL.

The intranasal administration was performed 20 minutes prior to a test session of a forced swim test.

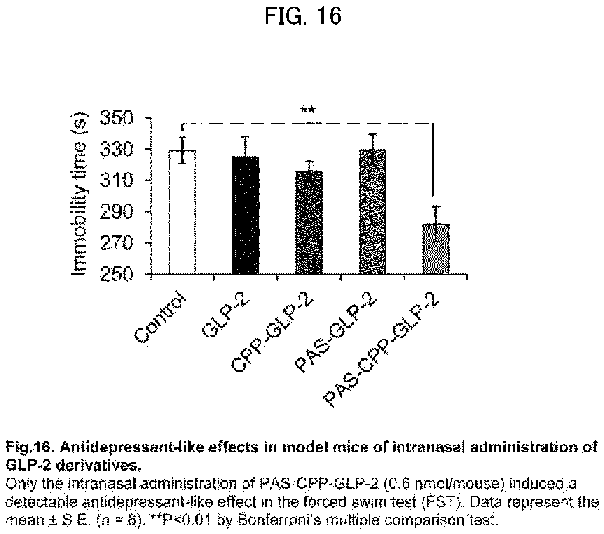

As shown in FIG. 16, the group administered with PAS-CPP-GLP-2 showed a significant decrease in immobility time as compared with the control group, indicating the expression of anti-depressant-like effects. The groups administered with PAS- GLP-2, CPP-GLP-2 or GLP-2 did not show a significant decrease in immobility time as compared with the control group, not indicating the expression of anti-depressant-like effects.

### <Example 16: Study on glycosylated GLP-1 derivative using amyloid β-induced neurodegenerative models>

### (Preparation of amyloid β-induced neurodegenerative models>

A solution of Aβ1-42, containing 1 mg/mL of amyloid β protein fragment (Aβ1-42), was prepared with normal saline and incubated for 4 days at 37°C. After anesthetizing the mice with isoflurane, the mice were administered with the Aβ1-42 solution (3 µL) by intraventricular administration. The administration was performed using a 50-µL syringe (Hamilton (R) GASTIGHT (R) syringe, 1700) and an intracerebral needle (28G x 3mm, Natsume Seisakusho) over 15 seconds in consideration of a pressure difference.

The neurodegenerative model mice were divided into the Vehicle group and the GLP-1 derivative group, and the Vehicle group was intranasally administered with PBS and the GLP-1 derivative group was intranasally administered with the PAS-CPP-GLP-1 derivative glycosylated at N terminus (11-sugar) used in Example 14 by the amount of 0.1 nmol/mouse or 1.0 nmol/mouse). The intranasal administration was performed once a day for 7 days. The intranasal administration was performed to the mice after anesthetizing the same using a micropipette of 2-20 µL. Specifically, the intranasal administration was performed by contacting a tip of the micropipette to a nasal cavity in a horizontal manner such that the solution was inhaled by spontaneous respiration, by the amount of 2 µL per nose cavity, 4 µL in total.

### (Preparation of frozen brain sections)

The brains of respective groups were isolated and subjected to infiltration fixation with 4% PFA at 4°C overnight. On the day following the isolation, the brains were substituted with 30% sucrose overnight (4°C). Thereafter, frozen sections of the hippocampal CA1 region, the hippocampal dentate gyrus and the cerebral cortex with a thickness of 30 µm were prepared using a cryostat (CM3050S; Leica Microsystems, WetZlar, Germany). For the purpose of comparison, frozen sections were prepared from the mice intranasally administered with PBS instead of intraventricular administration with Aβ1-42 solution.

### (Immunostaining of neurodegenerative cells)

The frozen sections were placed on a seal and dried. Thereafter, the sections were immersed in a basic alcohol solution for 5 minutes, subsequently in a 70% ethanol solution for 2 minutes, and subsequently in PBS for 2 minutes. The basic alcohol solution was prepared by mixing 20 mL of 5% sodium hydroxide aqueous solution with 80 mL of anhydrous ethanol. The sections were dried to a certain degree and immersed in 0.04% potassium permanganate solution for 3 minutes, and subsequently in PBS for 2 minutes. Thereafter, the sections were immersed in 0.0001% Fluoro-Jade B staining solution (FJB) and 2.5 µg/mL DAPI for 10 minutes, and then rinsed with purified water three times. The FJB solution was prepared with 0.1% acetic acid solution. The sections were dried and immersed in xylene for 1 minute. The sections were further dried and mounted on a slide glass using Fluoromount^{™}, and the fluorescence corresponding to the hippocampal CA1 region, the hippocampal dentate gyrus and the cerebral cortex was observed. The fluorescence intensity of green fluorescence, representing the neurodegenerative cells, was measured using Image J.

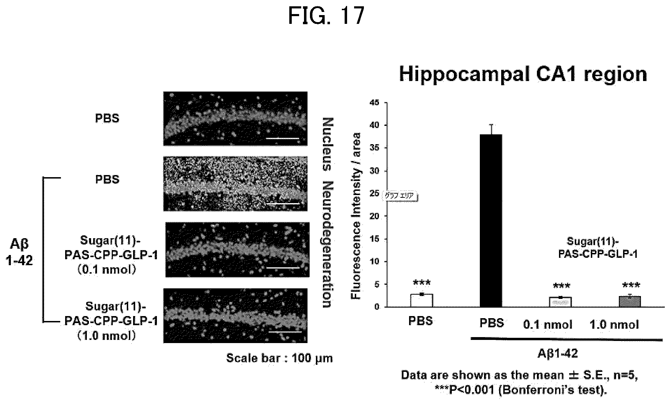

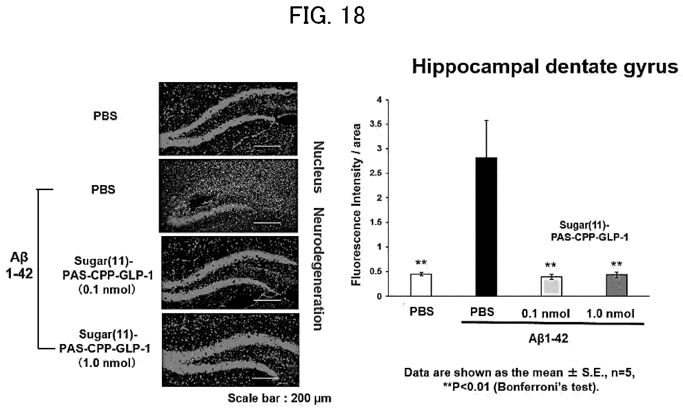

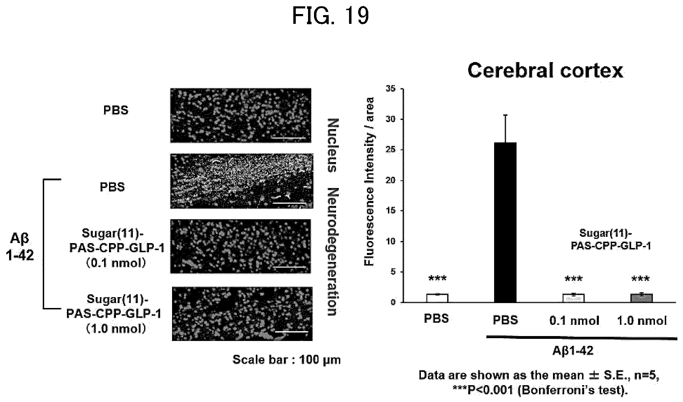

FIG. 17 shows the measurement results of the neurodegeneration in the hippocampal CA1 region, FIG. 18 shows the measurement results of the neurodegeneration in the hippocampal dentate gyrus, and FIG. 19 shows the measurement results of the neurodegeneration in the cerebral cortex.

As shown in FIG. 17, FIG. 18 and FIG. 19, the neurodegeneration of the model mice prepared using Aβ1-42 is significantly suppressed in the group administered with GLP-1 at each region of the hippocampal CA1 region, the hippocampal dentate gyrus and the cerebral cortex.

The disclosure of Japanese Patent Application No. 2022-180380 is incorporated herein by reference in its entirety. All publications, patent applications, and technical standards mentioned in the present specification are incorporated herein by reference to the same extent as if each individual publication, patent application, or technical standard was specifically and individually indicated to be incorporated by reference.

## Claims

1. A glycosylated neuropeptide derivative, comprising:
a neuropeptide sequence;
a central-nerve migration accelerating sequence that includes a cell-penetration accelerating sequence and an endosomal-escape accelerating sequence; and
a glycosylation molecule that includes a sugar chain.

2. The glycosylated neuropeptide derivative according to claim 1, further comprising a spacer sequence that is disposed between the neuropeptide sequence and the central-nerve migration accelerating sequence, wherein the glycosylation molecule is bound to the spacer sequence.

3. The glycosylated neuropeptide derivative according to claim 2, wherein the spacer sequence includes a lysine residue, and the glycosylation molecule is bound to the lysine residue.

4. The glycosylated neuropeptide derivative according to claim 1, wherein the glycosylation molecule further includes a linker.

5. The glycosylated neuropeptide derivative according to claim 4, wherein the linker includes an alkylene group with a carbon number of from 3 to 15.

6. The glycosylated neuropeptide derivative according to claim 1, wherein the glycosylation molecule is bound to a C-terminus side or an N-terminus side of the neuropeptide sequence.

7. The glycosylated neuropeptide derivative according to claim 1, wherein a number of monosaccharide residues per sugar chain is from 5 to 20.

8. The glycosylated neuropeptide derivative according to claim 1, wherein a number of amino acid residues of the neuropeptide sequence is 200 or less.

9. The glycosylated neuropeptide derivative according to claim 1, wherein the cell-penetration accelerating sequence is cationic.

10. The glycosylated neuropeptide derivative according to claim 1, wherein half or more of a total number of amino acid residues of the cell-penetration accelerating sequence are basic amino acid residues.

11. The glycosylated neuropeptide derivative according to claim 1, wherein the endosomal-escape accelerating sequence is an amino acid sequence selected from the group consisting of FFLIPKG, LILIG, FFG, FFFFG and FFFFFFG.

12. A pharmaceutical composition, comprising the glycosylated neuropeptide derivative according to any one of claim 1 to claim 11 as an active ingredient.

13. The pharmaceutical composition according to claim 12, used for therapy of a neuropsychiatric disorder or a neurodegenerative disorder.

14. The pharmaceutical composition according to claim 12, used for therapy of depression or dementia.

15. An intranasal/nasal drop formulation, comprising the glycosylated neuropeptide derivative according to any one of claim 1 to claim 11 as an active ingredient.

16. The intranasal/nasal drop formulation according to claim 15, used for therapy of a neuropsychiatric disorder or a neurodegenerative disorder.

17. The intranasal/nasal drop formulation according to claim 15, used for therapy of depression or dementia.

18. Use of the glycosylated neuropeptide derivative according to any one of claim 1 to claim 11 for intranasal/nasal drop administration.
